Europäisches Patentamt

European Patent Office

Office européen des brevets

(19)

(11) **EP 1 258 811 A1**

(12) **EUROPEAN PATENT APPLICATION**
published in accordance with Art. 158(3) EPC

(43) Date of publication:
**20.11.2002 Bulletin 2002/47**

(51) Int Cl.[7]: **G06F 17/00**, G01N 33/50

(21) Application number: **00977998.4**

(86) International application number:
**PCT/JP00/08402**

(22) Date of filing: **29.11.2000**

(87) International publication number:
**WO 01/040969 (07.06.2001 Gazette 2001/23)**

(84) Designated Contracting States:
**AT BE CH CY DE DK ES FI FR GB GR IE IT LI LU MC NL PT SE TR**

(30) Priority: **29.11.1999 JP 33856099**

(71) Applicant: **Riken**
**Wako-shi, Saitama 351-0198 (JP)**

(72) Inventor: **HAYASHIZAKI, Yoshihide,**
**c/o Riken Tsukuba Inst**
**Tsukuba-shi, Ibaraki 305-0074 (JP)**

(74) Representative: **Gervasi, Gemma, Dr.**
**NOTARBARTOLO & GERVASI Srl,**
**Corso di Porta Vittoria, 9**
**20122 Milano (IT)**

(54) **EXSON-INTRON JUNCTION DETERMINING DEVICE, GENETIC REGION DETERMINING DEVICE, AND DETERMINING METHOD FOR THEM**

(57)    The present invention provides a device and a method for efficiently determining an exon-intron junction with high accuracy. The device of the invention is useful for determining an exon-intron junction in a gene region of the genome. This device comprises an input part in which data on a cDNA of organism 1 and the corresponding gene region of organism 2 are input; an operation part in which two non-overlapped sequences i and j, each having at least 10 bases, are extracted from the gene region of organism 2, and, with respect to sequences i and j extracted, $s(i, j)$ defined by $s(i, j) = s(x, y_{ij}) - C\{(b_1 - j) + (i - a_2) - (B_1 - A_2)\}^2$ is calculated; a junction determination part in which a combination of sequences i and j that maximizes $s(i, j)$ is selected; and an output part in which the position of the exon-intron junction determined is output.

```
┌─────────────────────┐
│     INPUT PART      │
└─────────────────────┘
           │
           ▼
┌─────────────────────┐
│   OPERATION PART    │
└─────────────────────┘
           │
           ▼
┌─────────────────────┐
│      JUNCTION       │
│   DETERMINATION     │
│       PART          │
└─────────────────────┘
           │
           ▼
┌─────────────────────┐
│    OUTPUT PART      │
└─────────────────────┘
```

**FIG. I**

**Description**

BACKGROUND THE INVENTION

Field of the Invention

The present invention relates to a device for determining an exon-intron junction, to a device for determining a gene in the genome, specifically, a cDNA region, and to a method for the same.

Related Art

Grail, Grail 2 and Genscan have been known as programs for predicting exons in DNA sequences. It is possible to predict the nucleotide sequence of a part of a gene by these prediction programs, but it is still difficult to predict the nucleotide sequence or amino acid sequence of the whole genome. Furthermore, since these programs are based on a learning method by computers, time for prediction becomes longer as the data size of the nucleotide sequence increases. The exon prediction accuracy is approximately 70%, especially, the prediction accuracy of initiation codon involved in the formation of a protein in genes is approximately 40%.

On the other hand, the human genome project is now under way and a method for efficiently and highly accurately identifying human genes, specifically, cDNA sequences, on the basis of the data of the human genome is needed.

SUMMARY OF THE INVENTION

We have now found a method for efficiently identifying exon-intron junction(s) in a gene region of the genome with high accuracy. We have also found a method for determining, on the basis of the information on a full-length cDNA sequence of a first organism, homologous regions in an unknown gene region of a second organism.

An object of the present invention is to provide a device for efficiently predicting, identifying or determining an exon-intron junction in a gene region of the genome with high accuracy.

Another object of the present invention is to provide a device for efficiently predicting, identifying or determining a cDNA region of the genome with high accuracy.

A further object of the present invention is to provide a computer readable memory medium storing a program for efficiently predicting, identifying or determining an exon-intron junction in a gene region of the genome with high accuracy.

Yet another object of the present invention is to provide a computer readable memory medium storing a program for efficiently predicting, identifying or determining a cDNA region of the genome with high accuracy.

A still further object of the present invention is to provide a method for efficiently predicting, identifying or determining an exon-intron junction in a gene region of the genome with high accuracy.

Another object of the present invention is to provide a method for efficiently predicting, identifying or determining a cDNA region of the genome with high accuracy.

According to the first embodiment, there is provided a device for predicting, identifying or determining an exon-intron junction in a gene region of the genome, comprising:

an input part in which data on a full-length cDNA sequence of organism 1 or a part thereof (fragment AB) and the corresponding gene region of the genome of organism 2 (fragment ab) are input;

an operation part in which two non-overlapped sequences, each having at least 10 bases, are extracted from fragment ab, wherein the sequences present on the 5' side and the 3' side of fragment ab are represented by "i" and "j", respectively, and s(i, j) defined by the following equation is calculated with respect to sequences i and j extracted:

$$s(i, j) = s(x, yij) - C\{(b - j) + (i - a) - (B - A)\}^2 \qquad (I)$$

wherein

$$s(x, yij) = \max (v(k)) \qquad (II)$$

$$V(k) = \sum_{p=1}^{myij} M(k+p,p) \qquad \text{(III)} \qquad [k, ?]$$

(b - j) represents the number of bases between the 3' end of the gene region of organism 2 and the 5' end of sequence j,

(i - a) represents the number of bases between the 5' end of the gene region of organism 2 and the 3' end of sequence i,

(B - A) represents the number of bases in the cDNA of organism 1,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

a junction determination part in which a combination of sequences i and j that maximizes s(i, j) is selected; and

an output part in which the position of the exon-intron junction determined is output.

According to the second embodiment, there is provided a device for predicting, identifying or determining an exon-intron junction in a gene region of the genome, comprising:

an input part in which data on a full-length cDNA sequence of organism 1 or a part thereof (fragment AB) and the corresponding gene region of the genome of organism 2 (fragment ab) are input, wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, and regions A1A2 and B1B2 are homologous with regions a1a2 and b1b2, respectively;

an operation part in which two non-overlapped sequences, each having at least 10 bases, are extracted from a region between a1a2 and b1b2 in the gene region of the genome of organism 2, wherein the sequences present on the 5' end side and the 3' end side fragment ab are represented by "i" and "j", respectively, and s(i, j) defined by the following equation is calculated with respect to sequences i and j extracted:

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p,p) \qquad \text{(III)}$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq 10$,

mj represents the number of bases in sequence j and is $\geq 10$, and

myij represents the number of bases in sequence yij and is $\geq 20$;

a junction determination part in which a combination of sequences i and j that maximizes s(i, j) is selected; and

an output part in which the position of the exon-intron junction determined is output.

According to the third embodiment, there is provided a device for predicting, identifying or determining a cDNA region of the genome, comprising:

an input part in which data on a full-length cDNA sequence of organism 1 or a part thereof, data on the whole genome DNA sequence of organism 2 or a part thereof and a list of the positions of homologous regions between the cDNA sequence of organism 1 and the genome DNA sequence of organism 2 are input, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;

an operation part in which two non-overlapped sequences, each having at least 10 bases, are extracted from a region between each two neighboring homologous regions, in which region the sequences present on the 5' end side and the 3' end side are represented by "i" and "j", respectively, and s(i, j) defined by the following equation is calculated with respect to sequences i and j extracted from the region between each two neighboring homologous regions:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq 10$,

mj represents the number of bases in sequence j and is $\geq 10$, and

myij represents the number of bases in sequence yij and is $\geq 20$;

a junction determination part in which a combination of sequences i and j that maximizes s(i, j) is selected with respect to the region between each two neighboring homologous regions; and

an output part in which intron sequences are cut out from the genome DNA sequence of organism 2 according to the positions of the exon-intron junctions determined, the remaining sequences are connected, and the cDNA sequence of organism 2 is output.

According to the fourth embodiment, there is provided a device for predicting, identifying or determining a cDNA region of the genome, comprising:

an input part in which data on a full-length cDNA sequence of organism 1 or a part thereof and data on the whole genome DNA sequence of organism 2 or a part thereof are input;

a homology search part in which homologous regions in the genome DNA sequence of organism 2 that are homologous with the full-length cDNA sequence of organism 1 or a part thereof are searched;

a position list making part in which combinations of the homologous regions in the genome DNA sequence of organism 2 are made; combinations that cannot exist as cDNA sequences are removed from the combinations obtained; and a combination that gives the widest coverage on the genome DNA is selected from the remaining combinations, thereby making a list of the positions of the homologous regions, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;

an operation part in which two non-overlapped sequences, each having at least 10 bases, are extracted from a region between each two neighboring homologous regions, in which region the sequences present on the 5' end side and the 3' end side are represented by "i" and "j", respectively, and s(i, j) defined by the following equation is calculated with respect to sequences i and j extracted from the region between each two neighboring homologous regions:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

a junction determination part in which a combination of sequences i and j that maximizes s(i, j) is selected with respect to the region between each two neighboring homologous regions; and

an output part in which intron sequences are cut out from the genome DNA sequence of organism 2 according to the positions of the exon-intron junctions determined, the remaining sequences are connected, and the cDNA sequence of organism 2 is output.

According to the fifth embodiment, there is provided a computer readable memory medium storing a program for predicting, identifying or determining an exon-intron junction in a gene region of the genome, wherein the program executes the following instructions:

instructions for extracting two non-overlapped sequences, each having at least 10 bases, from a gene region of the genome of organism 2 (fragment ab) that corresponds to a full-length cDNA sequence of organism 1 or a part thereof (fragment AB), wherein the sequences present on the 5' side and the 3' side of fragment ab are represented by "i" and "j", respectively;

instructions for calculating, with respect to sequences i and j extracted, s(i, j) defined by the following equation:

$$s(i, j) = s(x, yij) - C\{(b - j) + (i - a) - (B - A)\}^2 \qquad (I)$$

wherein

$$s(x, yij) = \max (v(k)) \qquad (II)$$

$$V(k) = \sum_{p=1}^{myij} M(k+p, p) \qquad (III)$$

(b - j) represents the number of bases between the 3' end of the gene region of organism 2 and the 5' end of sequence j,
(i - a) represents the number of bases between the 5' end of the gene region of organism 2 and the 3' end of sequence i,
(B - A) represents the number of bases in the cDNA of organism 1,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20; and
instructions for selecting a combination of sequences i and j that maximizes s(i, j), thereby determining the position of the exon-intron junction.

According to the sixth embodiment, there is provided a computer readable memory medium storing a program for predicting, identifying or determining an exon-intron junction in a gene region of the genome, wherein the program executes the following instructions:

instructions for extracting two non-overlapped sequences, each having at least 10 bases, from a region between a1a2 and b1b2 in a gene region of the genome of organism 2 (fragment ab) that corresponds to a full-length cDNA sequence of organism 1 or a part of it (fragment AB), wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, regions A1A2 and B1B2 are homologous with regions a1a2 and b1b2, respectively, and the sequences present on the 5' side and the 3' side of fragment ab are represented by "i" and "j", respectively;
instructions for calculating, with respect to sequences i and j extracted, s(i, j) defined by the following equation:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region blb2 and the 5' end of sequence j,

(i - a2) represents the number of bases between the 5' end of region ala2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq 10$,

mj represents the number of bases in sequence j and is $\geq 10$, and

myij represents the number of bases in sequence yij and is $\geq 20$; and

instructions for selecting a combination of sequences i and j that maximizes s(i, j), thereby determining the position of the exon-intron junction.

According to the seventh embodiment, there is provided a computer readable memory medium storing a program for predicting, identifying or determining a cDNA region of the genome, wherein the program executes the following instructions:

instructions for extracting two non-overlapped sequences, each having at least 10 bases, from a region between each two neighboring homologous regions on the genome of organism 2 on the basis of data on a full length cDNA sequence of organism 1 or a part thereof, data on the whole genome DNA sequence of organism 2 or a part thereof and a list of the positions of homologous regions between the cDNA sequences of organism 1 and the genome DNA sequence of organism 2, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., regions A1A2, B1B2, ..... are homologous with regions a1a2, b1b2, ....., respectively, and the sequences present on the 5' side and the 3' side of fragment ab are represented by "i" and "j", respectively;

instructions for calculating, with respect to sequences i and j extracted from the region between each two neighboring homologous regions, s(i, j) defined by the following equation:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq 10$,

mj represents the number of bases in sequence j and is $\geq 10$, and

myij represents the number of bases in sequence yij and is $\geq 20$;

instructions for selecting, with respect to the region between each two neighboring homologous regions, a combination of sequences i and j that maximizes s(i, j), thereby determining the positions of exon-intron junction(s); and instructions for cutting intron sequence(s) out from the genome DNA sequence of organism 2 according to the positions of the exon-intron junction(s) determined, and connecting the remaining pieces to determine the cDNA sequence of organism 2.

According to the eighth embodiment, there is provided a computer readable memory medium storing a program for predicting, identifying or determining a cDNA region of the genome, wherein the program executes the following instructions:

instructions for searching homologous regions in the genome DNA sequence of organism 2 that are homologous with a full-length cDNA sequence of organism 1 or a part thereof on the basis of data on the full-length cDNA sequence of organism 1 or a part thereof and data on the whole genome DNA sequence of organism 2 or a part thereof;
instructions for making combinations of the homologous regions in the genome DNA sequence of organism 2;
instructions for removing, from the combinations obtained, combinations that cannot exist as cDNA sequences;
instructions for selecting, from the combinations obtained, a combination that gives the widest coverage on the genome DNA sequence of organism 2, thereby making a list of the positions of the homologous regions, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;
instructions for selecting two non-overlapped sequences, each having at least 10 bases, from a region between each two neighboring homologous regions, in which region the sequences present on the 5' end side and the 3' end side are represented by "i" and "j", respectively;
instructions for calculating, with respect to sequences i and j extracted from the region between each two neighboring homologous regions, s(i, j) defined by the following equation:

$$s(i, j) = s(x, yij) - C\{(b1 - j) + (i - a2) - (B1 - A2)\}^2 \qquad (Ia)$$

wherein

$$s(x, yij) = \max(v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is ≥ 10,
mj represents the number of bases in sequence j and is ≥ 10, and
myij represents the number of bases in sequence yij and is ≥ 20;
instructions for selecting, with respect to the region between each two neighboring homologous regions, a combination of sequences i and j that maximizes s(i, j), thereby determining the positions of exon-intron junction(s); and
instructions for cutting intron sequence(s) out from the genome DNA sequence of organism 2 according to the positions of the exon-intron junction(s) determined, and connecting the remaining pieces to determine the cDNA sequence of organism 2.

According to the ninth embodiment, there is provided a method for predicting, identifying or determining an exon-intron junction in a gene region of the genome, comprising the steps of:

preparing data on a full-length cDNA sequence of organism 1 or a part thereof (fragment AB) and the corresponding gene region of the genome of organism 2 (fragment ab);

extracting two non-overlapped sequences, each having at least 10 bases, from fragment ab, wherein the sequences present on the 5' side and the 3' side of fragment ab are represented by "i" and "j", respectively;

calculating, with respect to sequences i and j extracted, s(i, j) defined by the following equation :

$$s(i, j) = s(x, yij) - C\{(b - j) + (i - a) - (B - A)\}^2 \qquad (I)$$

wherein

$$s(x, yij) = \max (v(k)) \qquad (II)$$

$$V(k) = \sum_{p=1}^{myij} M(k+p, p) \qquad (III)$$

(b - j) represents the number of bases between the 3' end of the gene region of organism 2 and the 5' end of sequence j,

(i - a) represents the number of bases between the 5' end of the gene region of organism 2 and the 3' end of sequence i,

(B - A) represents the number of bases in the cDNA of organism 1,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

selecting a combination of sequences i and j that maximizes s(i, j); and

determining the position of the exon-intron junction.

According to the tenth embodiment, there is provided a method for predicting, identifying or determining an exon-intron junction in a gene region of the genome, comprising the steps of:

preparing data on a full-length cDNA sequence of organism 1 or a part thereof (fragment AB) and the corresponding gene region of the genome of organism 2 (fragment ab), wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, and regions A1A2 and B1B2 are homologous with regions a1a2 and b1b2, respectively;

extracting two non-overlapped sequences, each having at least 10 bases, from a region between a1a2 and b1b2 in the gene region of the genome of organism 2, wherein the sequences present on the 5' end side and the 3' end side fragment ab are represented by "i" and "j", respectively;

calculating, with respect to sequences i and j extracted, s(i, j) defined by the following equation:

$$s(i, j) = s(x, yij) - C\{(b1 - j) + (i - a2) - (B1 - A2)\}^2 \qquad (Ia)$$

wherein

$$s(x, yij) = max (v(k)) \qquad (II)$$

$$\begin{array}{c} myij \\ V(k)=\Sigma M(k+p,p) \qquad (III) \\ p=1 \end{array}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20;
selecting a combination of sequences i and j that maximizes s(i, j); and
determining the position of the exon-intron junction.

According to the eleventh embodiment, there is provided a method for predicting, identifying or determining a cDNA region of the genome, comprising the steps of:

preparing an input part in which data on a full-length cDNA sequence of organism 1 or a part thereof, data on the whole genome DNA sequence of organism 2 or a part thereof and a list of the positions of homologous regions between the cDNA sequence of organism 1 and the genome DNA sequence of organism 2, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;
extracting two non-overlapped sequences, each having at least 10 bases, from a region between each two neighboring homologous regions, in which region the sequences present on the 5' end side and the 3' end side are represented by "i" and "j", respectively;
calculating ,with respect to sequences i and j extracted from the region between each two neighboring homologous regions, s(i, j) defined by the following equation :

$$s(i, j) = s(x, yij) - C\{(b1 - j) + (i - a2) - (B1 - A2)\}^2 \qquad (Ia)$$

wherein

$$s(x, yij) = max (v(k)) \qquad (II)$$

$$\begin{array}{c} myij \\ V(k)=\Sigma M(k+p,p) \qquad (III) \\ p=1 \end{array}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

selecting a combination of sequences i and j that maximizes s(i, j) with respect to the region between each two neighboring homologous regions;

determining the position of the exon-intron junction;

cutting out intron sequences from the genome DNA sequence of organism 2 according to the positions of the exon-intron junctions determined; and

connecting the remaining sequences, thereby determining the cDNA sequence of organism 2.

According to the twelfth embodiment, there is provided a method for predicting, identifying or determining a cDNA region of the genome, comprising the steps of:

preparing data on a full-length cDNA sequence of organism 1 or a part thereof and data on the whole genome DNA sequence of organism 2 or a part thereof;

searching homologous regions in the genome DNA sequence of organism 2 that are homologous with the full-length cDNA sequence of organism 1 or a part thereof;

making combinations of the homologous regions in the genome DNA sequence of organism 2;

removing combinations that cannot exist as cDNA sequences from the combinations obtained;

selecting a combination that gives the widest coverage on the genome DNA from the remaining combinations, thereby making a list of the positions of the homologous regions, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;

extracting two non-overlapped sequences, each having at least 10 bases, from a region between each two neighboring homologous regions, in which region the sequences present on the 5' end side and the 3' end side are represented by "i" and "j", respectively;

calculating, with respect to sequences i and j extracted from the region between each two neighboring homologous regions,s(i, j) defined by the following equation:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v( k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq 20$;

selecting a combination of sequences i and j that maximizes s(i, j) with respect to the region between each two neighboring homologous regions;

determining the position of the exon-intron junction;

cutting out intron sequences from the genome DNA sequence of organism 2 according to the positions of the exon-intron junctions determined; and

connecting the remaining sequences, thereby determining the cDNA sequence of organism 2.

According to the device of the first or second embodiment of the present invention, it is possible to efficiently predict, identify or determine an exon-intron junction in a gene region of the genome with high accuracy.

According to the device of the third or fourth embodiment of the present invention, it is possible to efficiently predict, identify or determine a cDNA region of the genome with high accuracy and the devices of the embodiments are particularly advantageous in precisely determining an entire gene region, not a part of the gene region.

According to the memory medium of the fifth or sixth embodiment of the present invention, it is possible to efficiently predict, identify or determine an exon-intron junction in a gene region of the genome with high accuracy.

According to the memory medium of the seventh or eighth embodiment of the present invention, it is possible to efficiently predict, identify or determine a cDNA region of the genome with high accuracy and the mediums of the embodiments are particularly advantageous in precisely determining an entire gene region, not a part of the gene region.

According to the method of the ninth or tenth embodiment of the present invention, it is possible to efficiently predict, identify or determine an exon-intron junction in a gene region of the genome with high accuracy.

According to the method of the eleventh or twelfth embodiment of the present invention, it is possible to efficiently predict, identify or determine a cDNA region of the genome with high accuracy and the methods of the embodiments are particularly advantageous in precisely determining an entire gene region, not a part of the gene region.

BRIEF DESCRIPTION OF THE DRAWINGS

Fig. 1 shows the first and second embodiments (the determination of an exon-intron junction) and the third embodiment (the determination of a cDNA region, characterized in inputting a list of homologous regions).

Fig. 2 shows the third embodiment further comprising a memory part that is connected to the operation part.

Fig. 3 shows the third embodiment further comprising an end part determination part that is provided after the junction determination part.

Fig. 4 shows the third embodiment further comprising a memory part that is connected to the operation part, and an end part determination part that is provided after the junction determination part.

Fig. 5 shows the fourth embodiment (the determination of a cDNA region, characterized in having a homology search part).

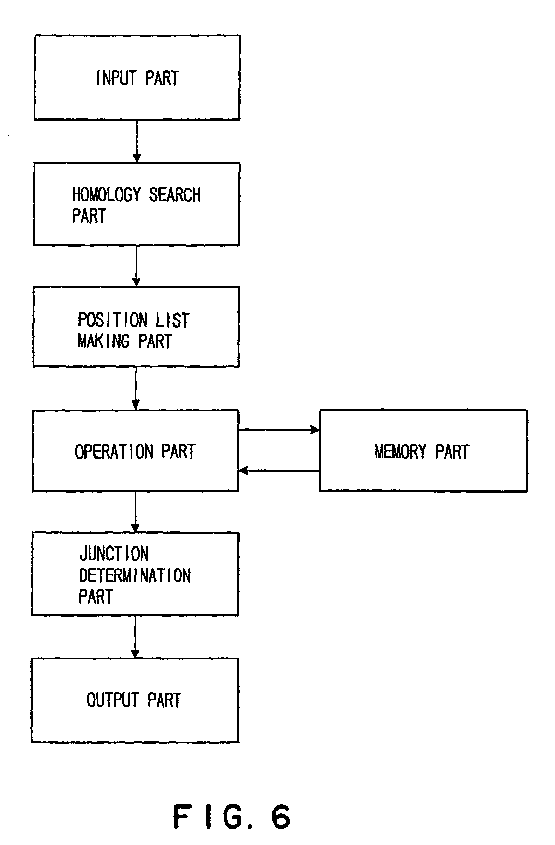

Fig. 6 shows the fourth embodiment further comprising a memory part that is connected to the operation part.

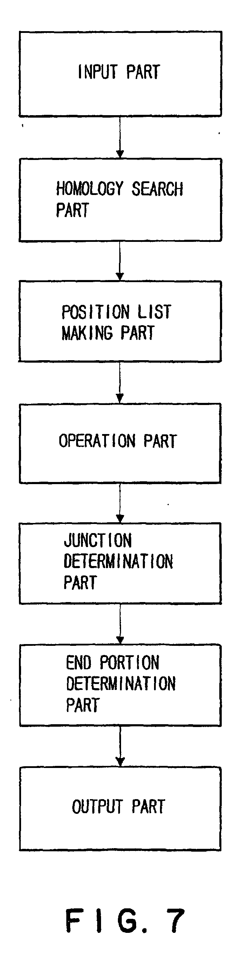

Fig. 7 shows the fourth embodiment further comprising an end part determination part that is provided after the junction determination part.

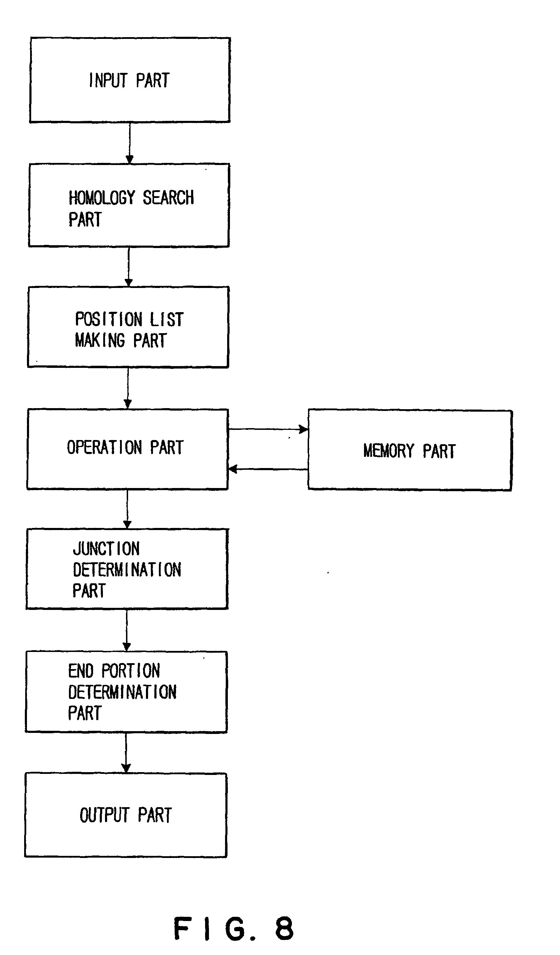

Fig. 8 shows the fourth embodiment further comprising a memory part that is connected to the operation part, and an end part determination part that is provided after the junction determination part.

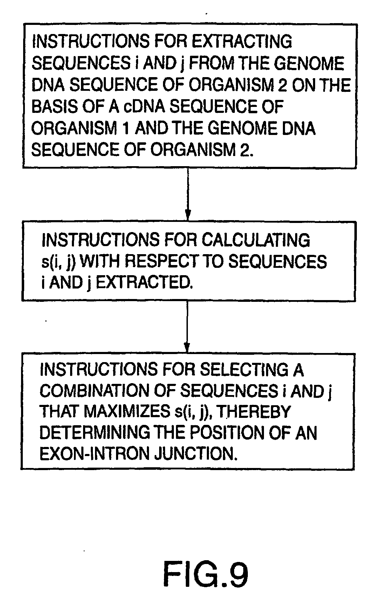

Fig. 9 shows the fifth and sixth embodiments (the determination of an exon-intron junction).

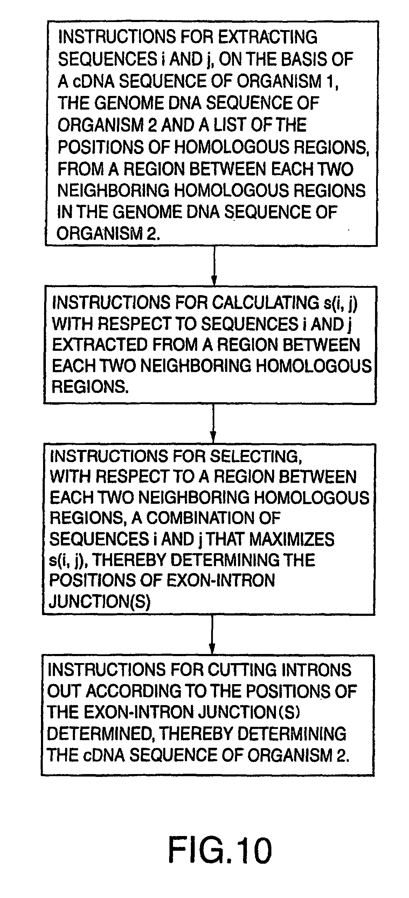

Fig. 10 shows the seventh embodiment (the determination of a cDNA region, characterized in inputting a list of homologous regions).

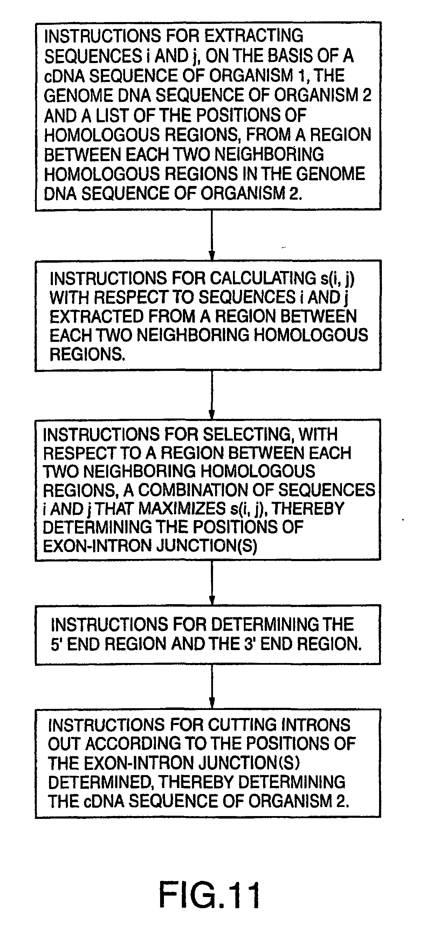

Fig. 11 shows the seventh embodiment further comprising instructions for determining end parts after the instructions for determining junctions.

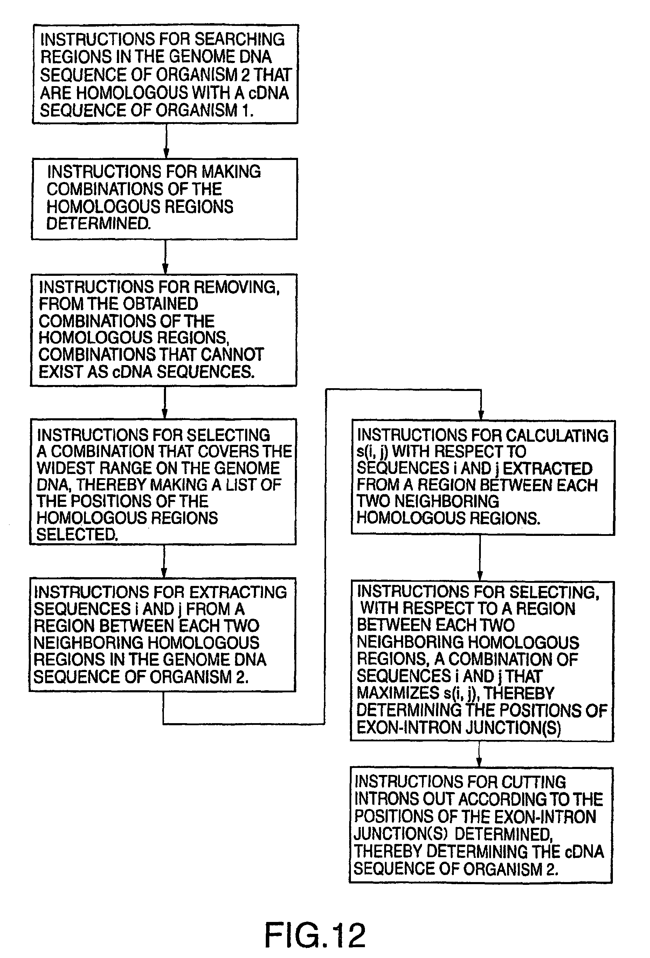

Fig. 12 shows the eighth embodiment (the determination of a cDNA region, characterized in having instructions for doing the homology search).

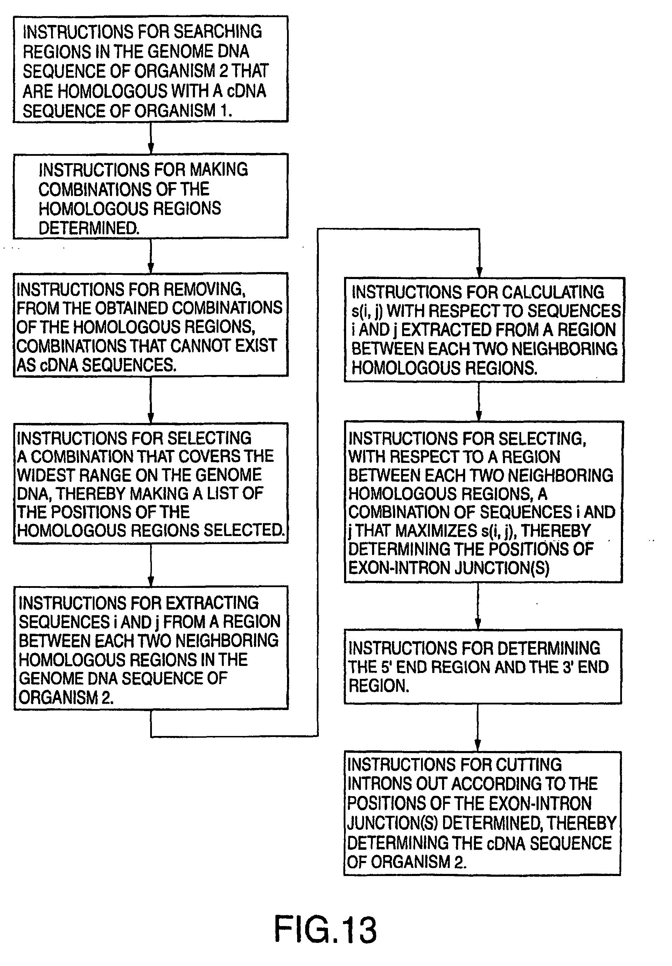

Fig. 13 shows the eighth embodiment further comprising instructions for determining end parts after the instructions for determining junctions.

Fig. 14 shows the ninth and tenth embodiments (the determination of an exon-intron junction).

Fig. 15 shows the eleventh embodiment (the determination of a cDNA region, characterized in inputting a list of homologous regions).

Fig. 16 shows the eleventh embodiment further comprising the step of determining end parts after the step of determining junctions.

Fig. 17 shows the twelfth embodiment (the determination of a cDNA region, characterized in having the step of doing the homology search).

Fig. 18 shows the twelfth embodiment further comprising the step of determining end parts after the step of determining junctions.

Fig. 19 shows the relationship between the cDNA sequence of organism 1 and the genome DNA sequence of organism 2.

Fig. 20 shows the relationship between a cDNA sequence of organism 1 and the genome DNA sequence of organism 2, where regions A1A2 and B1B2 are homologous with regions a1a2 and b1b2, respectively, and sequences i and j are selected in accordance with the GT-AG rule,

Fig. 21 shows an example of the combination of the homologous regions determined, indicating that four homologous regions are found in the genome DNA of organism 2. I, II, III and IV represents homologous regions.

Fig. 22 is an NS chart more specifically describing the instructions of the third embodiment of the present invention, where the list of splice site candidates, that is, junction candidates, in the respective homologous regions (1 to N) will be used in the instructions shown in Fig. 23.

Fig. 23 is an NS chart more specifically describing the instructions of the third embodiment of the present invention, where the number of splice site candidates on the 5' end side of each homologous region I (I = 1 to N) is represented by $n^5$ (I), the number of splice site candidates on the 3' end side of the same is represented by $n^3$ (I), the positions of the splice site candidates on the 5' end side are represented by $m^5$ (I, j) (j = 1 to $n^5$ (I)), and the positions of the splice site candidates on the 3' end side are represented by $n^3$ (I, i) (i = 1 to $n^3$ (I)).

Fig. 24 shows the homology search that is carried out on the genome DNA sequence of organism 2 on the basis of the cDNA sequence of organism 1, where two types of homologous regions are found and four homologous regions are found in the genome DNA of organism 2.

Fig. 25 is a perspective view of a computer that is used with respect to a memory medium storing a program for determining an exon-intron junction or a program for determing a cDNA region of a genome.

Fig. 26 is a block diagram showing the hardware constitution of the computer shown in Fig. 25.

DETAILED DESCRIPTION OF THE INVENTION

First and Second Embodiments

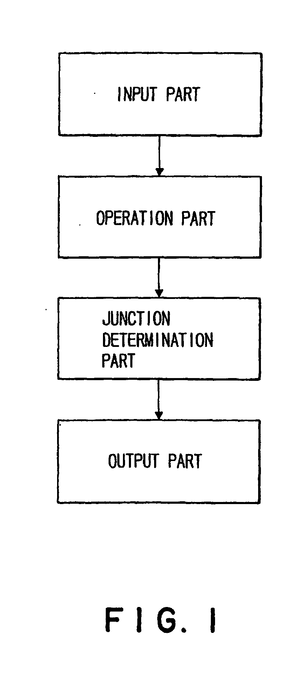

First and second embodiments of the present invention provide devices for identifying an exon-intron junction. The devices according to these two embodiments of the invention are as shown in Fig. 1. Specifically, these devices may be computer-based devices, that is, computer systems.

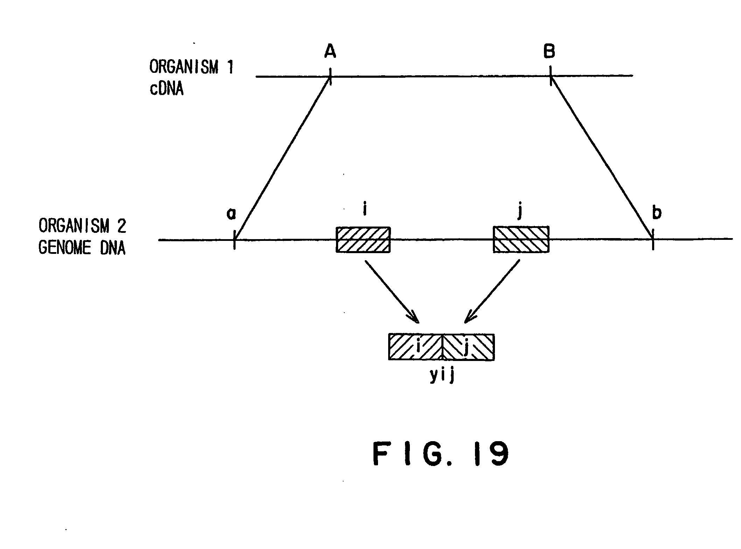

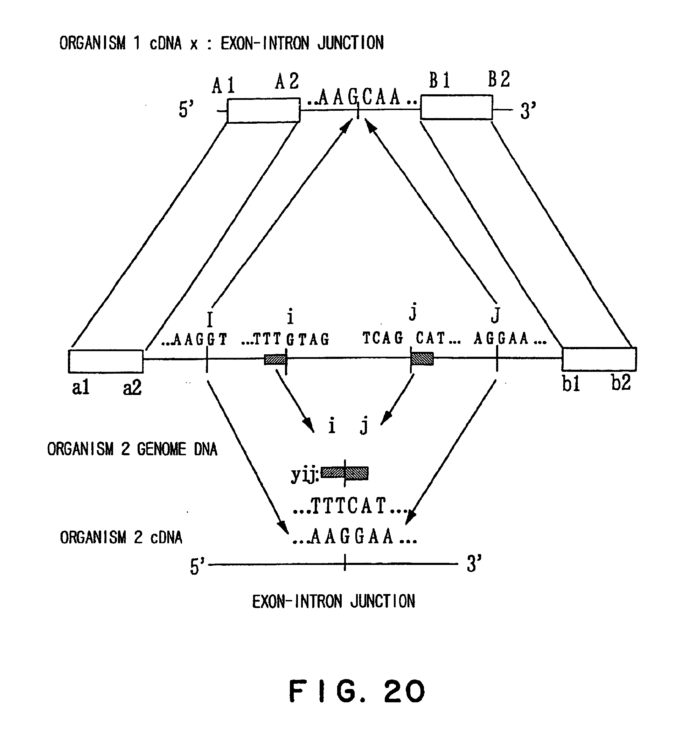

Firstly, in the input part, a full-length cDNA sequence of organism 1 or a part thereof (fragment AB) and the corresponding gene region of the genome of organism 2 (fragment ab) are input. This process is common to the first and second embodiments. The second embodiment is, however, different from the first embodiment in that the cDNA sequence of organism 1 or a part thereof and the gene region of the genome of organism 2 have, at their end parts, homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively. The relationship between the cDNA sequence of organism 1 and the genome DNA sequence of organism 2 are shown in Figures 19 and 20.

Organisms 1 and 2 may be selected so that close relation can be found between the two organisms in terms of the existence and/or homology of genes; for example, organisms 1 and 2 can be eukaryotes, specifically, mammals. More specifically, combinations of the two organisms are such that organism 1 is a mouse and organism 2 is a fly and that organism 1 is a fly and organism 2 is a human, respectively. In the case where the two organisms are both mammals, possible combinations are such that organism 1 is a mouse and organism 2 is a human and that organism 1 is a human and organism 2 is a mouse.

In the second embodiment, fragment ab is present between the above-described two homologous regions, and it is preferable that the two homologous regions exist side by side. In the case where fragment ab is present between the two homologous regions that exist side by side, there is a high possibility that one intron exists between these homologous regions. Fig. 20 shows a case where two homologous regions exist side by side and no other homologous regions exist between them.

In the operation part, two non-overlapped sequences, each containing at least 10 (e.g., 10 to 30), preferably at least 20 bases, are selected from the genome of organism 2, specifically, from fragment ab in the first embodiment, from the region between a1a2 and b1b2 in the second embodiment. Sequences that exist on the genome of organism 2 on its 5' end side and 3' end side are represented by "i" and "j", respectively (see Figs. 19 and 20). Preferably, two sequences, each having 20 base pairs, are selected.

Sequences i and j can be selected in accordance with the GT-AG rule (Mount, S.M., Nucleic Acid Res., 10, 459-472 (1982)).

Further, in the operation part, s(i, j), a function of sequences i and j, is calculated.

s(x, yij) included in equations (I) and (Ia) is calculated by using the following equation:

$$s(x, yij) = \max(v(k)) \qquad (II)$$

wherein v(k) is calculated by using the following equation:

$$V(k) = \sum_{p=1}^{myij} M(k+p, p) \qquad (III)$$

The calculation of s(x, yij) can be explained by taking an example where x is aagctggagactctct and yij is ggaga. In this case, the following matrix is obtained.

```
                     →x
              a a g c t g g a g a c t c t c t
         ↓  g 0 0 1 0 0 1 1 0 1 0 0 0 0 0 0
       yij  g 0 0 1 0 0 1 1 0 1 0 0 0 0 0 0
            a 1 1 0 0 0 0 0 1 0 1 0 0 0 0 0
            g 0 0 1 0 0 1 1 0 1 0 0 0 0 0 0
            a 1 1 0 0 0 0 0 1 0 1 0 0 0 0 0
         k =            1 2 3 4 5 6 7 8 9 10 11 12
```

M represents a matrix of x and yij, where M(a, b) = 1 when a base in position "a" for x is the same base as in position "b" for yij, and M(a, b) = 0 when a base in position "a" for x is not the same base as in position "b" for yij.

For example, when k = 2, the scores of those parts indicated by ● in the following matrix are calculated:

```
                     →x
              a a g c t g g a g a c t c t c t
         ↓  g 0 ● 1 0 0 1 1 0 1 0 0 0 0 0 0
       yij  g 0 0 ● 0 0 1 1 0 1 0 0 0 0 0 0
            a 1 1 0 ● 0 0 0 1 0 1 0 0 0 0 0
            g 0 0 1 0 ● 1 1 0 1 0 0 0 0 0 0
            a 1 1 0 0 0 ● 0 1 0 1 0 0 0 0 0
         k =            1 2 3 4 5 6 7 8 9 10 11 12
```

The values of v(k) are as follows: v(1) = 0, v(2) = 1, v(3) = 2, v(4) = 2, v(5) = 1, v(6) = 5, v(7) = 1, v(8) = 2, v(9) = 1, v(10) = 0, v(11) = 0, v(12) = 0. Therefore, s(x, yij) = max{0, 1, 2, 2, 1, 5, 1, 2, 1, 0, 0, 0}, and thus s(x, yij) = 5.

v(k) is preferably v'(k) that is defined by the following equation:

$$V'(k)=\sum_{p=1}^{myij} M(k+p,p)+\max(\sum_{p=1}^{myij} M(k-n+p,p)\times 0.5; n=-6\sim 6) \qquad (IV)$$

Even if deletion or addition of a base/bases takes place in the arrangement x or yij, it is possible to obtain a veritable maximum value by adding the correction term "max(ΣM(k-n+p, p) × 0.5; n = -6 to 6" to the equation (III), thereby smoothing the value of v(k), wherein n represents the number of gaps in the overlapped region and is preferably from -1 to 1. In this case, the value of v'(k) is equal to the sum of the value of v(k) and a half of the value that is greater one of the values of the two neighboring terms of v(k).

In equation (I), C is a proportionality constant. It is possible to determine C so that the prediction accuracy by the method according to the present invention will be maximum by preparing a plurality of combinations of the cDNA of organism 1 and that of organism 2, provided that the two cDNA are of the same type and that their full-length sequences are already known, and combinations of the above cDNAs and the genome of organism 2 comprising the cDNA which genome is clearly determined. Specifically, C may be from 0 to 10, preferably 0.5.

In equation (I), the value of myij is equal to the sum of the values of mi and mj. myij is an integer of 20 or more (e.g., 20 to 60), preferably 40 or more.

In the junction determination part, a combination of sequences i and j that maximizes the value of s (i, j) defined by equation (I) is selected. In the output part, the position of the exon-intron junction that has been determined according to the combination of sequences i and j selected is output.

Third Embodiment

The third embodiment of the present invention provides a device for identifying a cDNA region of the genome. The device according to this embodiment of the invention is as shown in Fig. 1. Specifically, this device may be a computer-based device, that is, a computer system.

Firstly, in the input part, data on a full-length cDNA sequence of organism 1 or a part thereof, data on the whole genome DNA sequence of organism 2 or a part thereof and a list of positions of homologous regions between the cDNA sequence of organism 1 and the genome DNA sequence of organism 2 are input. The list of the positions of homologous regions can be obtained by carrying out the homologugy search as described later in detail. Organisms 1 and 2 may be selected in the same manner as in the aforementioned first and second embodiments.

In the operation part, two non-overlapped sequences, each having at least 10 bases, are extracted from a region between each two neighboring homologous regions on the basis of the list of the positions of homologous regions that has already been input. If the region between each two neighboring homologous regions exists in the number of two or more, junction candidates are extracted from the respective regions.

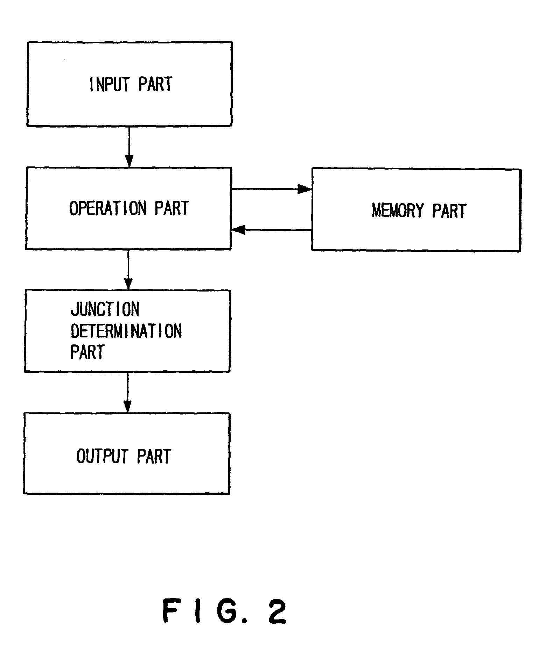

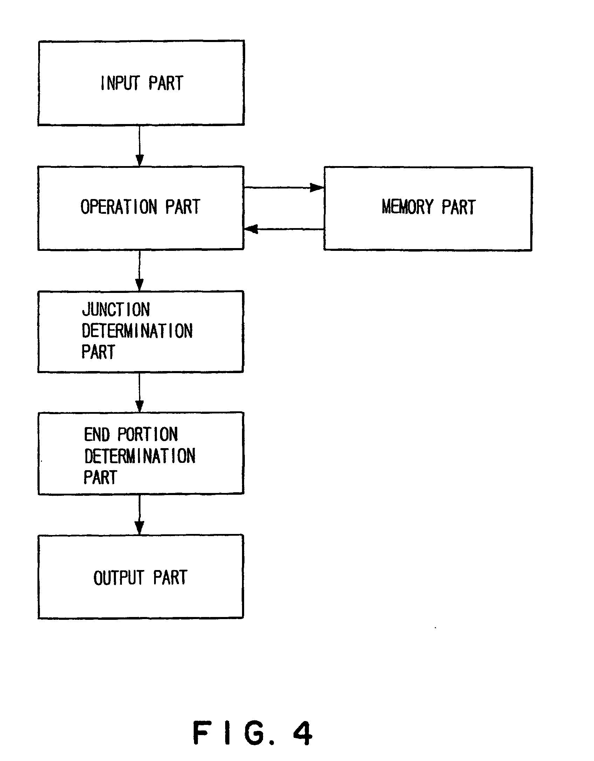

In the third embodiment, the device may further comprise a memory part in which junction candidates that have been extracted in the operation part are temporarily stored (Figs. 2 and 4). In the operation part, s(i, j) is calculated with respect to each one of the junction candidates in a certain region; in the junction determination part, a favorable junction is selected according to the values of s(i, j) obtained by calculation. After a junction in one region is determined, junction candidates in another region are extracted in the same way; s(i, j) is then calculated; the step of determining junctions is repeated.

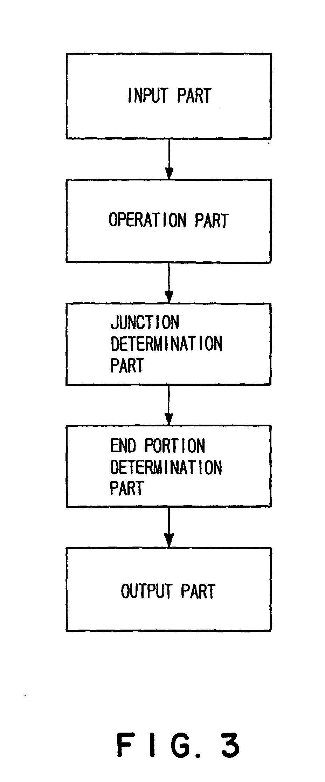

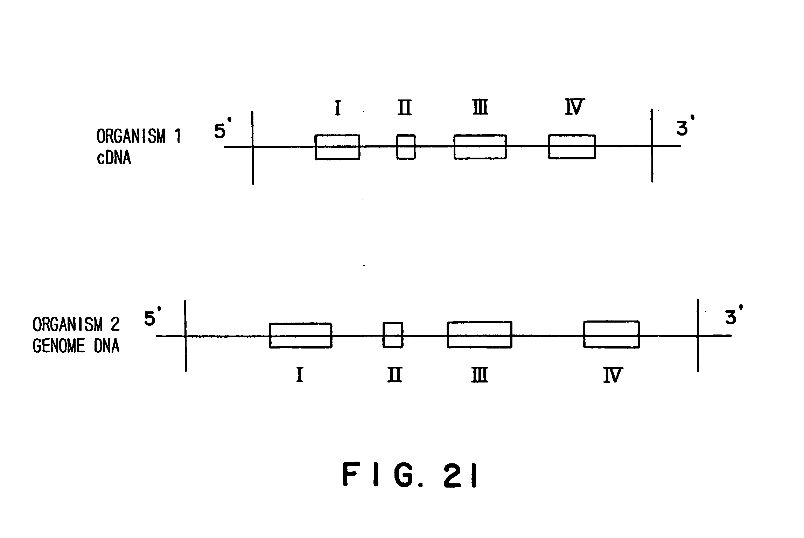

After exon-intron junctions are determined, 5' and 3' ends of the cDNA of organism 2 may be, if necessary, determined in the end part determination part by determining a gene region that exists 5'-upstream of the homologous region located on the very 5' end side of the genome DNA of organism 2, for example, region I in Fig. 21, and a gene region that exists 3'-downstream of the homologous region located on the very 3' end side of the same, for example, region IV in Fig. 21(Figs. 3 and 4). The 5' and 3' ends of the cDNA sequence of organism 2 can be determined by finding homologous regions having the same length as in the 5' end side and 3' end side of the cDNA of organism 1, for example, regions I and IV in Fig. 21, and eliminating base call errors and the like so that the cDNA of organism 1 and that of organism 2 can have the same length.

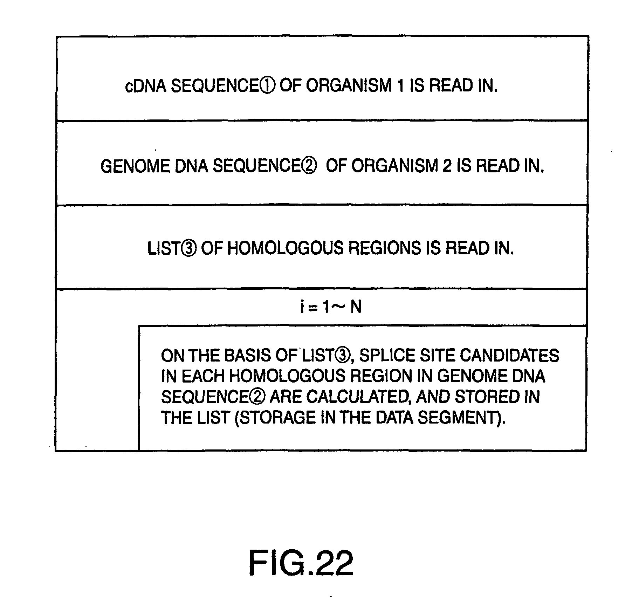

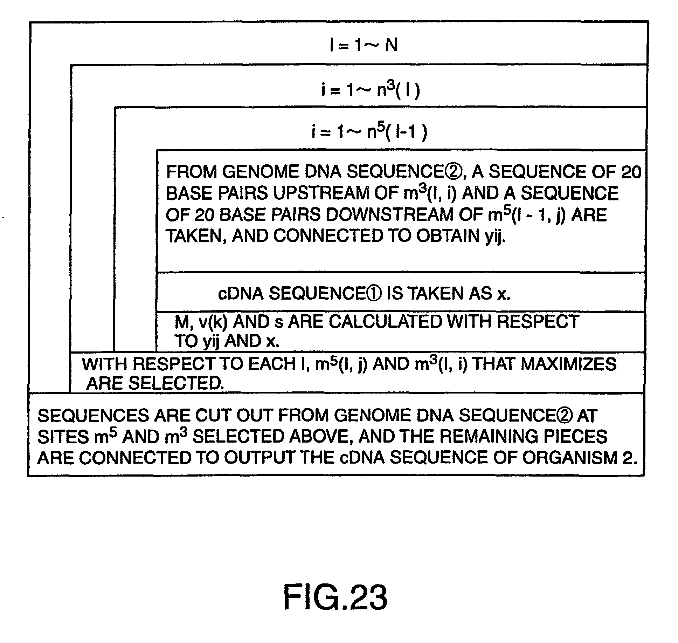

The NS charts shown in Figs. 22 and 23 more specifically describe data processing that is executed by the device of the third embodiment.

By using the device for identifying cDNA according to the third embodiment of the present invention, it is possible to determine, on the basis of the full-length cDNA sequence of a first organism, the full-length cDNA sequence of a second organism.

Fourth Embodiment

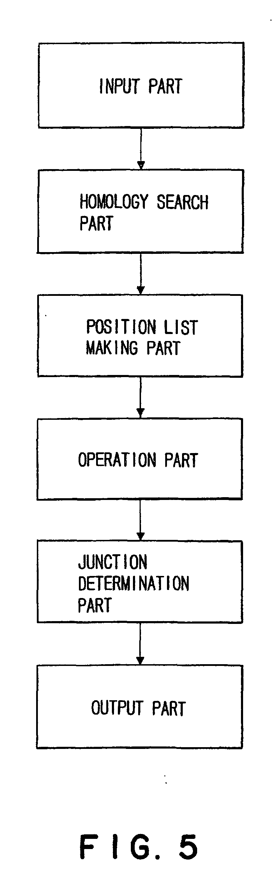

The fourth embodiment of the present invention provides a device for identifying the cDNA region of a genome. The device according to this embodiment of the invention is as shown in Fig. 5. Specifically, this device may be a computer-based device, that is, a computer system.

The fourth embodiment is the same as the third embodiment, except that the input part in the third embodiment is replaced with the input part, homology search part and position list making part.

Firstly, in the input part, data on a full-length cDNA sequence of organism 1 or a part thereof and data on the whole genome DNA sequence of organism 2 or a part thereof are input. Organisms 1 and 2 may be selected in the same manner as in the previous embodiments.

Next, in the homology search part, regions that are homologous with the full-length cDNA sequence of organism 1 or a part thereof are searched for the genome DNA sequence of organism 2. The homology search may be carried out with a probability of $10^{-50}$ or less, preferably $10^{-100}$ or less, more preferably $10^{-200}$ or less.

The homology search part may be a search system selected from BLAST, LALIGN, ALIGN and FASTA. Alternatively, the homology search part may be a search system that is connected to the above search system by means of a telecommunication line or the like.

In the position list making part, combinations are made with respect to the homologous regions in the genome DNA sequence of organism 2, determined by searching homologous regions in the genome DNA sequence of organism 2 that are homologous with the full-length cDNA sequence of organism 1 or a part thereof. Specifically, combinations of the homologous regions are made, considering as to whether the combinations can exist or not. If homologous regions exist in the number of q, combinations of the homologous regions can be obtained in the number of $2^q$.

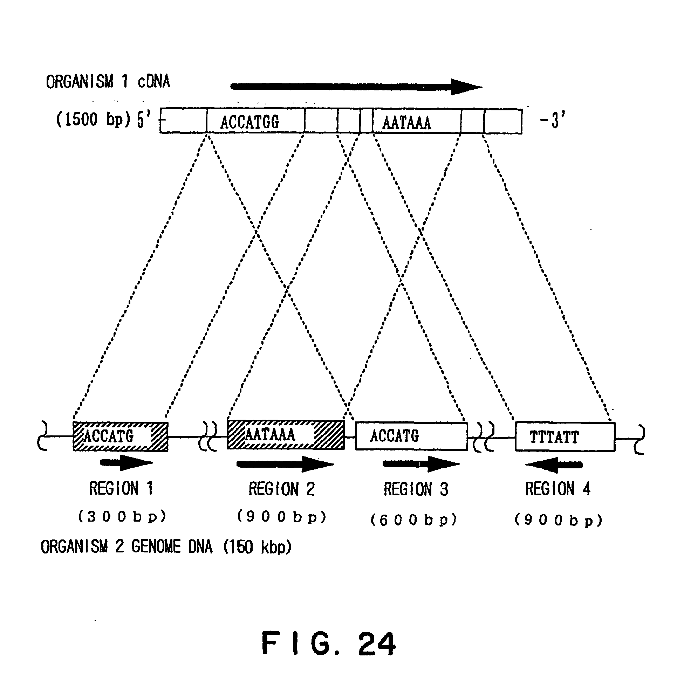

Assuming that two homologous regions were found by carrying out the homology search between the cDNA sequence of organism 1 and the genome DNA sequence of organism 2, it will be explained how to make combinations of the homologous regions. In the case where four homologous regions are found in organism 2 as shown in Fig. 24, the following 16 combinations of the homologous regions can be obtained.

| Combination | Region 1 | Region 2 | Region 3 | Region 4 | Coverage (bp) |
|:---:|:---:|:---:|:---:|:---:|:---:|
| (1) | 1 | 0 | 0 | 0 | 300 |
| (2) | 0 | 1 | 0 | 0 | 900 |
| (3) | 1 | 1 | 0 | 0 | 1200 |
| (4) | 0 | 0 | 1 | 0 | 600 |
| (5) | 1 | 0 | 1 | 0 | NG |
| (6) | 0 | 1 | 1 | 0 | NG |
| (7) | 1 | 1 | 1 | 0 | NG |
| (8) | 0 | 0 | 0 | 1 | 900 |
| (9) | 1 | 0 | 0 | 1 | NG |
| (10) | 0 | 1 | 0 | 1 | NG |
| (11) | 1 | 1 | 0 | 1 | NG |
| (12) | 0 | 0 | 1 | 1 | NG |
| (13) | 1 | 0 | 1 | 1 | NG |
| (14) | 0 | 1 | 1 | 1 | NG |
| (15) | 1 | 1 | 1 | 1 | NG |
| (16) | 0 | 0 | 0 | 0 | NG |
| NG: a combination that cannot exist. | | | | | |

Also, in the position list making part, those combinations that cannot exist as cDNA sequences are removed from the combinations obtained. The combinations that cannot exist as cDNA sequences are as follows:

- a combination in which homologous regions in organism 1 that correspond to two or more homologous regions in organism 2 are the same (e.g., combinations (5) and (7));
- a combination in which the order of two or more homologous regions in organism 2 is opposite to that of the corresponding homologous regions in organism 1 (e.g., combinations (6) and (7)); and
- a combination in which the directions of two or more homologous regions in organism 2 are inverted (e.g., combinations (9) to (15)).

EP 1 258 811 A1

In addition, combinations that cannot exist as cDNA sequences also include such a combination that a plurality of homologous regions is located 30 bp - 30 kbp apart (e.g., 5 kbp to 30 kbp apart in the case of higher organisms). The number of bases can specifically be determined so that the total length of the bases will be shorter than the mean distance between genes estimated from the density of genes present in the genome of organism 2 (one gene per 30 kbp in higher organisms) and longer than the minimum length of introns.

Furthermore, in the position list making part, a combination that gives the widest coverage on the genome DNA is selected from the combinations obtained, thereby making a list of the positions of the homologous regions selected. In the above example, combination (3) is selected as a favorable combination of the homologous regions.

In the operation part, the position list of the homologous regions made in the position list making part is input, and junction candidates are extracted on the basis of the data on the cDNA sequence of organism 1 and the genome DNA sequence of organism 2, which have already been input.

Processing in the operation part, junction determination part, and output part can be executed in the same manner as in the third embodiment.

Fifth, Sixth, Seventh, and Eighth Embodiments

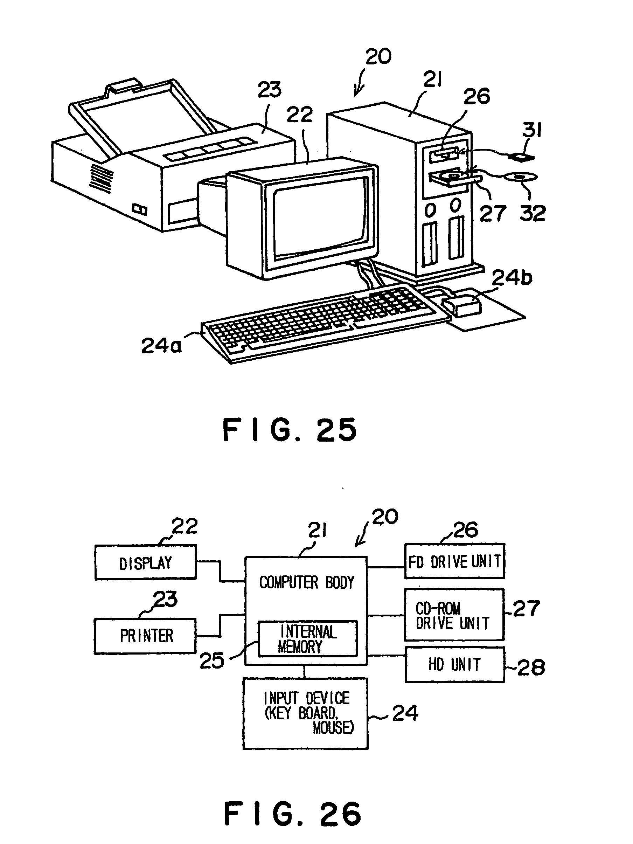

The input part, operation part, junction determination part and output part, and, if necessary, the memory part and end part determination part in the first, second and third embodiments, as well as the input part, homology search part, position list making part, operation part, junction determination part and output part, and, if necessary, the memory part and end part determination part in the fourth embodiment are provided as program modules that are executed by computer 20 as shown in Fig. 25. A program for determining an exon-intron junction or a cDNA region of the genome having the above modules is stored in a memory medium such as a floppy disc or CD-ROM (Compact Disk-Read Only Memory), and read out by computer 20 to determine an exon-intron junction or a cDNA region of the genome. These programs may be distributed through telecommunication lines (including radio communication lines), for example, through the Internet (carrier wave). Further, the programs may also be distributed through telecommunication lines, for example, through the Internet,while being coded, modulated or compressed. The programs may be distributed while being stored in a memory medium.

As shown in Fig. 25, computer 20 comprises computer body 21 placed in a housing such as a mini-tower, display 22 such as a CRT (Cathode Ray Tube) display, printer 23 that serves as a recording/output device, key board 24a and mouse 24b as an input device, floppy disk drive unit 26 by which the information recorded in floppy disk 31, memory medium, is read out, and CD-ROM drive unit 27 by which the information recorded in CD-ROM 32, memory medium, is read out.

The block diagram in Fig. 26 shows the above-described construction of the computer. As shown in this figure, the housing in which computer body 21 is placed further includes internal memory 25 composed of RAM (Random Access Memory) or the like, and an external storage such as hard disk unit 28 or the like. Floppy disk (recording medium) 31 in which the program for determining an exon-intron junction or a CDNA region of the genome has been stored is inserted into the slot of floppy disk drive unit 26 as shown in Fig. 25, whereby the program is installed in computer body 21 through the prescribed instructions. The memory medium in which the program of the present invention is stored is not limited to floppy disk 31; CD-ROM 32, inner memory 25, hard disk unit 28 or the like, or even an MO (Magnet Optical) disk, an optical disk, a DVD (Digital Versatile Disk) or the like, which is not shown in the figure, can be used as a memory medium.

Ninth, Tenth, Eleventh, and Twelfth Embodiments

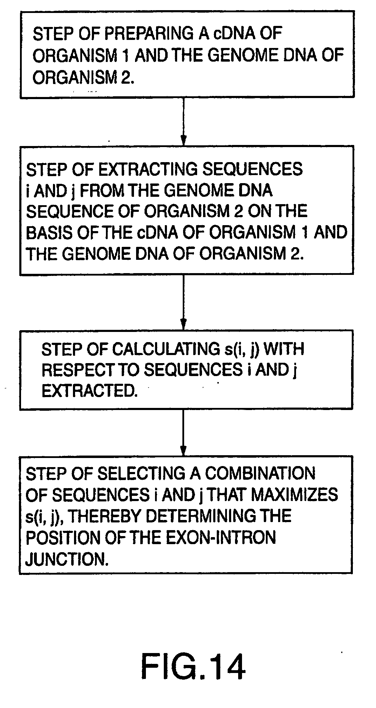

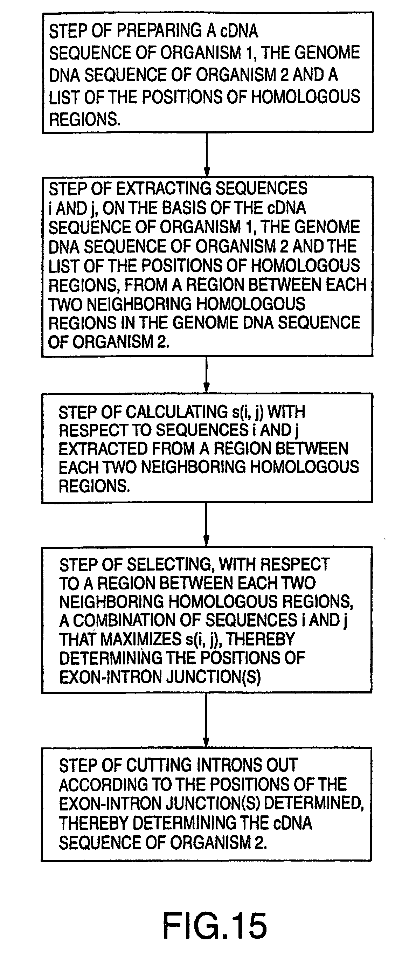

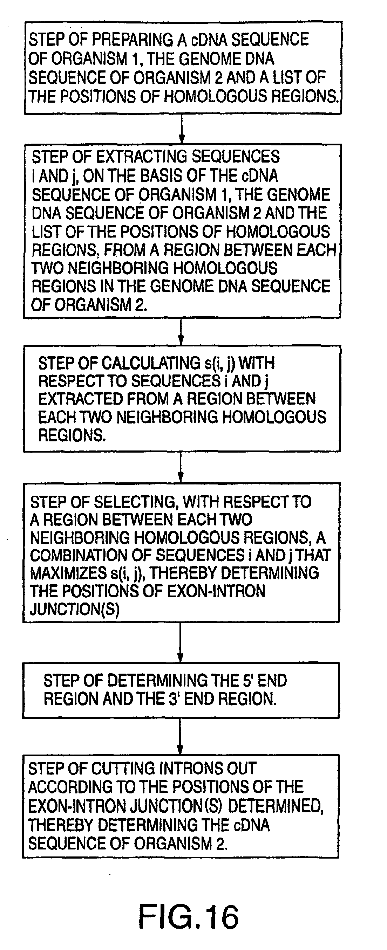

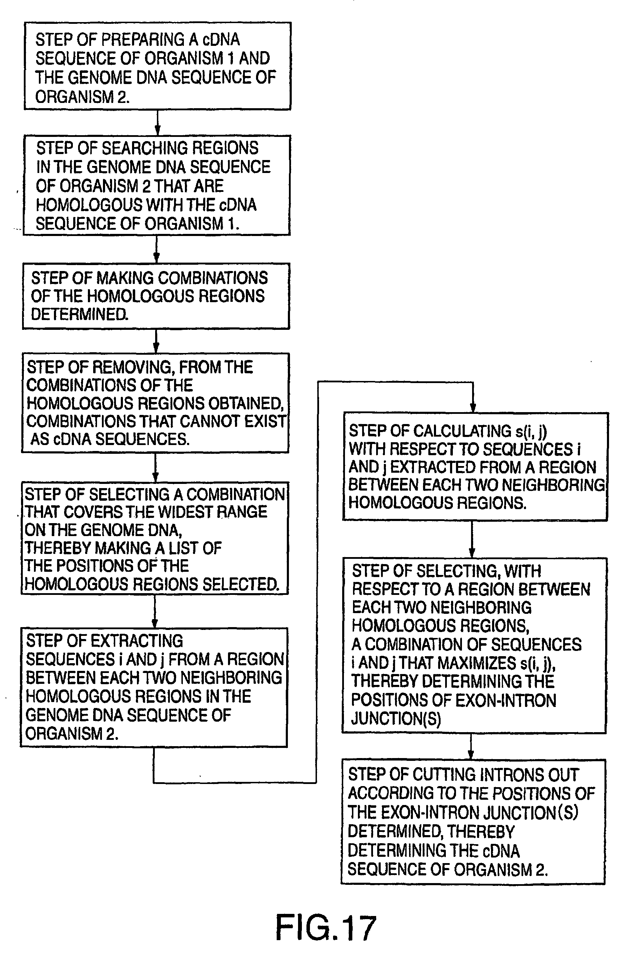

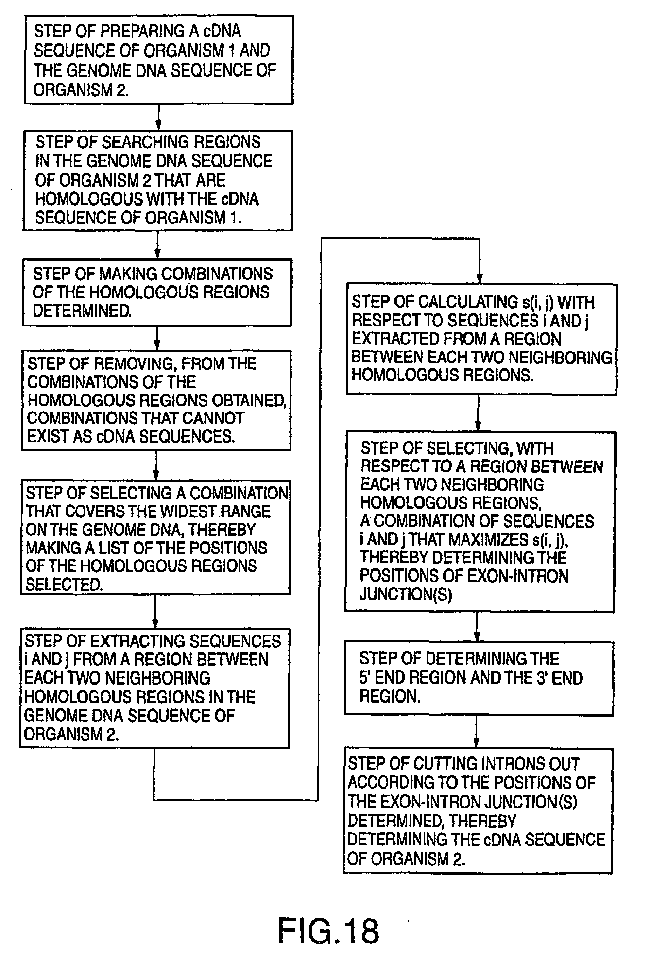

The methods according to the present invention respectively comprise the steps that are excuted by the devices of the first, second, third, and fourth embodiments. Flow charts of these embodiments are shown in Figs. 14 to 18.

EXAMPLE

The following is an example that according to the present invention, a cDNA region of the human genome was determined based on a mouse cDNA.

Twenty mouse cDNAs were taken from the brain, renal cell and 18-day embryo of a C57BL/6 mouse, and sequenced.

The homology search was conducted using BLAST. The probability of the homology search was set at $10^{-50}$.

From combinations of homologous regions obtained, the following combinations that cannot exist were removed:

- a combination in which homologous regions in organism 1 that correspond to two or more homologous regions in organism 2 are the same;
- a combination in which the order of two or more homologous regions in organism 2 is opposite to that of the corresponding homologous regions in organism 1;
- a combination in which the directions of two or more homologous regions in organism 2 are inverted; and
- a combination in which a plurality of homologous regions are at least 5 kbp apart.

Exon-intron junctions were detected using the following equation:

$$s(i, j) = s(x, yij) - 0.5 \times \{(b1 - j) + (i - a2) - (B1 - A2)\}^2 \qquad (I)$$

wherein

$$s(x, yij) = \max(v'(k)) \qquad (II)$$

$$V'(k) = \sum_{p=1}^{myij} M(k+p,p) + \max(\sum_{p=1}^{myij} M(k-n+p,p) \times 0.5; n = -6 \sim 6) \qquad (VI)$$

mi = 20, mj = 20, and myij = 40.

Sequences i and j were selected in accordance with the GT-AG rule.

The results were as shown in Tables 1 and 2.

TABLE 1

| | | Predicted Human Protein | | | | Mouse Protein | |
|---|---|---|---|---|---|---|---|
| Human Protein | aa[a] | Global Identity (%) | aa[b] | Partial Identity (%) | aa[c] | Global Identity (%) | aa[d] |
| GI4502098 | 298 | 100.0 | 298 | 100.0 | 298 | 89.6 | 296 |
| AF039689 | 303 | 85.8 | 262 | 99.2 | 262 | 96.7 | 304 |
| HUMCIPA | 480 | 87.6 | 510 | 94.7 | 473 | 80.2 | 437 |
| AF098668 | 231 | 95.1 | 243 | 100.0 | 231 | 99.1 | 231 |
| HS560B094 | 141 | 100.0 | 141 | 100.0 | 141 | 94.3 | 137 |
| D87292 | 297 | 100.0 | 297 | 100.0 | 297 | 90.9 | 297 |
| HSU63810 | 339 | 90.1 | 353 | 94.6 | 336 | 44.8 | 167 |
| HUMCG22 | 193 | 76.6 | 238 | 100.0 | 187 | 70.1 | 244 |
| HSU72513 | 144 | 73.6 | 108 | 98.1 | 108 | 38.2 | 128 |
| HSA011497 | 211 | 74.9 | 158 | 100.0 | 158 | 92.4 | 211 |
| HSCALT | 172 | 67.4 | 116 | 100.0 | 116 | 95.9 | 172 |
| HUMRAN | 200 | 95.5 | 194 | 100.0 | 191 | 93.6 | 203 |
| GI4507370 | 292 | 46.6 | 151 | 82.5 | 120 | 61.8 | 189 |
| GI4502600 | 277 | 100.0 | 277 | 100.0 | 277 | 53.1 | 181 |
| GI4506996 | 314 | 71.0 | 223 | 100.0 | 223 | 69.1 | 223 |
| HSU65581 | 407 | 62.4 | 287 | 100.0 | 241 | 55.3 | 240 |
| HSU82808 | 491 | 48.5 | 297 | 85.5 | 297 | 42.7 | 283 |
| HUMZC48G12 | 123 | 98.4 | 122 | 98.4 | 122 | 79.7 | 123 |
| AF043341 | 91 | 100.0 | 91 | 100.0 | 91 | 80.2 | 91 |
| AF042164 | 70 | 84.3 | 70 | 85.5 | 69 | 56.2 | 80 |

Table 1 shows the comparison between the mouse proteins and the human proteins determined. In this table, "a" represents the number of amino acid residues on the human protein; "b" represents the number of amino acid residues on the human protein predicted; "c" represents the number of amino acid residues aligned between the human protein and the predicted human protein; and "d" represents the number of amino acid residues on the mouse protein. The partial sequence identity was calculated by using LALIGN (Huang, X., Hardison, R.C., and Miller, W., Comput. Appl. Biosci., 6, 373-381, 1990).

Of the 20 proteins, 5 human full-length proteins were accurately determined using the method according to the present invention. On the other hand, only 3 human full-length proteins were precisely determined when Genscan was used, and no full-length proteins could be accurately determined when Grail 2 was used (data not shown).

TABLE 2

| DNA sequence prediction accuracy and false positive rate using the method of the present invention | | | |
|---|---|---|---|
| Prediction accuracy: 85.1% (= 19036 bp/22374 bp) | | | |
| False positive: 14.9% (= 3338 bp/22374 bp) | | | |
| Amino acid sequence prediction accuracy and false positive rate using the method of the present invention, Genscan or Grail 2 | | | |
| | Present Method | Genscan | Grail 2 |
| Prediction accuracy | 83.3% (= 3697 aa/4436 aa) | 51.0% (= 4854 aa/9517 aa) | 77.9% (= 3204 aa/4111 aa) |
| False positive | 16.7% (= 739 aa/4436 aa) | 49.0% (= 4663 aa/9517 aa) | 22.1% (= 907 aa/4111 aa) |

Table 2 shows the comparison between the prediction accuracy for the method of the present invention and that for Genscan and Grail 2. In this table, the accuracy (%) is obtained by dividing the number of amino acid residues accurately sequenced by the total number of amino acid residues sequenced; and the false positive (%) is obtained by dividing the number of amino acid residues incorrectly sequenced by the total number of amino acid residues sequenced.

The accuracy rate of the method according to the present invention is as high as 83.3%, while the false positive rate is only 16.7%. The accuracy rate and false positive rate of the method according to the present invention are thus high and low, respectively, as compared with Genscan and of Grail 2.

**Claims**

1. A device for predicting, identifying or determining an exon-intron junction in a gene region of the genome, comprising:

an input part in which data on a full-length cDNA sequence of organism 1 or a part thereof (fragment AB) and the corresponding gene region of the genome of organism 2 (fragment ab) are input;
an operation part in which two non-overlapped sequences, each having at least 10 bases, are extracted from fragment ab, wherein the sequences present on the 5' side and the 3' side of fragment ab are represented by "i" and "j", respectively, and s(i, j) defined by the following equation is calculated with respect to sequences i and j extracted:

$$s(i, j) = s(x, yij) - C\{(b - j) + (i - a) - (B - A)\}^2 \qquad (I)$$

wherein

$$s(x, yij) = \max (v(k)) \qquad (II)$$

$$V(k) = \sum_{p=1}^{myij} M(k+p, p) \qquad (III)$$

(b - j) represents the number of bases between the 3' end of the gene region of organism 2 and the 5' end of sequence j,

(i - a) represents the number of bases between the 5' end of the gene region of organism 2 and the 3' end of sequence i,

(B - A) represents the number of bases in the cDNA of organism 1,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

a junction determination part in which a combination of sequences i and j that maximizes s(i, j) is selected; and

an output part in which the position of the exon-intron junction determined is output.

2. A device for predicting, identifying or determining an exon-intron junction in a gene region of the genome, comprising:

an input part in which data on a full-length cDNA sequence of organism 1 or a part thereof (fragment AB) and the corresponding gene region of the genome of organism 2 (fragment ab) are input, wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, and regions A1A2 and B1B2 are homologous with regions a1a2 and b1b2, respectively;

an operation part in which two non-overlapped sequences, each having at least 10 bases, are extracted from a region between a1a2 and b1b2 in the gene region of the genome of organism 2, wherein the sequences present on the 5' end side and the 3' end side fragment ab are represented by "i" and "j", respectively, and s(i, j) defined by the following equation is calculated with respect to sequences i and j extracted:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

a junction determination part in which a combination of sequences i and j that maximizes s(i, j) is selected; and

an output part in which the position of the exon-intron junction determined is output.

3. A device for predicting, identifying or determining a cDNA region of the genome, comprising:

an input part in which data on a full-length cDNA sequence of organism 1 or a part thereof, data on the whole genome DNA sequence of organism 2 or a part thereof and a list of the positions of homologous regions between the cDNA sequence of organism 1 and the genome DNA sequence of organism 2 are input, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively; an operation part in which two non-overlapped sequences, each having at least 10 bases, are extracted from a region between each two neighboring homologous regions, in which region the sequences present on the 5' end side and the 3' end side are represented by "i" and "j", respectively, and s(i, j) defined by the following equation is calculated with respect to sequences i and j extracted from the region between each two neighboring homologous regions:

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v( k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20;
a junction determination part in which a combination of sequences i and j that maximizes s(i, j) is selected with respect to the region between each two neighboring homologous regions; and
an output part in which intron sequences are cut out from the genome DNA sequence of organism 2 according to the positions of the exon-intron junctions determined, the remaining sequences are connected, and the cDNA sequence of organism 2 is output.

4. A device for predicting, identifying or determining a cDNA region of the genome, comprising:

an input part in which data on a full-length cDNA sequence of organism 1 or a part thereof and data on the whole genome DNA sequence of organism 2 or a part thereof are input;
a homology search part in which homologous regions in the genome DNA sequence of organism 2 that are homologous with the full-length cDNA sequence of organism 1 or a part thereof are searched;
a position list making part in which combinations of the homologous regions in the genome DNA sequence of organism 2 are made; combinations that cannot exist as cDNA sequences are removed from the combinations obtained; and a combination that gives the widest coverage on the genome DNA is selected from the remaining combinations, thereby making a list of the positions of the homologous regions, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;

an operation part in which two non-overlapped sequences, each having at least 10 bases, are extracted from a region between each two neighboring homologous regions, in which region the sequences present on the 5' end side and the 3' end side are represented by "i" and "j", respectively, and s(i, j) defined by the following equation is calculated with respect to sequences i and j extracted from the region between each two neighboring homologous regions:

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20;
a junction determination part in which a combination of sequences i and j that maximizes s(i, j) is selected with respect to the region between each two neighboring homologous regions; and
an output part in which intron sequences are cut out from the genome DNA sequence of organism 2 according to the positions of the exon-intron junctions determined, the remaining sequences are connected, and the cDNA sequence of organism 2 is output.

5. The device according to claim 4, wherein the combinations that cannot exist as cDNA sequences in the position list making part are as follows:

- a combination in which homologous regions in organism 1 that correspond to two or more homologous regions in organism 2 are the same;
- a combination in which the order of two or more homologous regions in organism 2 is opposite to that of the corresponding homologous regions in organism 1; and
- a combination in which the directions of two or more homologous regions in organism 2 are inverted.

6. The device according to claim 4, wherein the homology search is made with a probability of not more than $10^{-50}$ in the homology search part.

7. The device according to claim 4, wherein the homology search part is a search system selected from BLAST, LALIGN, ALIGN and FASTA, or a search system connected to the search system by means of a telecommunication line.

8. The device according to claim 3 or 4, further comprising an end part determination part in which a region that exists 5'-upstream of the homologous region located on the very 5' end side of the genome DNA sequence of organism 2, and a region that exists 3'-downstream of the homologous region located on the very 3' end side of the same are determined.

9. The device according to any of claims 1 to 8, wherein v(k) in the operation part is represented by the following equation.

$$V'(k)=\sum_{p=1}^{myij} M(k+p,p)+\max(\sum_{p=1}^{myij} M(k-n+p,p)\times 0.5; n=-6\sim 6) \qquad (VI)$$

10. The device according to any of claims 1 to 9, wherein sequences i and j are extracted in accordance with the GT-AG rule in the junction determination part.

11. The device according to any of claims 1 to 10, wherein mi is 20, mj is 20, and myij is 40.

12. The device according to any of claims 1 to 11, wherein organisms 1 and 2 closely relate to each other in terms of the existence and/or homology of genes.

13. The device according to claim 12, wherein organisms 1 and 2 are eukaryotes.

14. The device according to claim 12, wherein organisms 1 and 2 are mammals.

15. The device according to claim 14, wherein organism 1 is a mouse and organism 2 is a human.

16. The device according to claim 14, wherein organism 1 is a human and organism 2 is a mouse.

17. A computer readable memory medium storing a program for predicting, identifying or determining an exon-intron junction in a gene region of the genome, wherein the program executes the following instructions:

instructions for extracting two non-overlapped sequences, each having at least 10 bases, from a gene region of the genome of organism 2 (fragment ab) that corresponds to a full-length cDNA sequence of organism 1 or a part thereof (fragment AB), wherein the sequences present on the 5' side and the 3' side of fragment ab are represented by "i" and "j", respectively;
instructions for calculating, with respect to sequences i and j extracted, s(i, j) defined by the following equation:

$$s(i, j) = s(x, yij) - C\{(b - j) + (i - a) - (B - A)\}^2 \qquad (I)$$

wherein

$$s(x, yij) = \max (v(k)) \qquad (II)$$

$$V(k)=\sum_{p=1}^{myij} M(k+p,p) \qquad (III)$$

(b - j) represents the number of bases between the 3' end of the gene region of organism 2 and the 5' end of sequence j,
(i - a) represents the number of bases between the 5' end of the gene region of organism 2 and the 3' end of sequence i,
(B - A) represents the number of bases in the cDNA of organism 1,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20; and

instructions for selecting a combination of sequences i and j that maximizes s(i, j), thereby determining the position of the exon-intron junction.

**18.** A computer readable memory medium storing a program for predicting, identifying or determining an exon-intron junction in a gene region of the genome, wherein the program executes the following instructions:

instructions for extracting two non-overlapped sequences, each having at least 10 bases, from a region between a1a2 and b1b2 in a gene region of the genome of organism 2 (fragment ab) that corresponds to a full-length cDNA sequence of organism 1 or a part of it (fragment AB), wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, regions A1A2 and B1B2 are homologous with regions a1a2 and b1b2, respectively, and the sequences present on the 5' side and the 3' side of fragment ab are represented by "i" and "j", respectively;

instructions for calculating, with respect to sequences i and j extracted, s(i, j) defined by the following equation:

$$s(i, j) = s(x, yij) - C\{(b1 - j) + (i - a2) - (B1 - A2)\}^2 \qquad \text{(Ia)}$$

wherein

$$s(x, yij) = \max(v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v( k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20; and

instructions for selecting a combination of sequences i and j that maximizes s(i, j), thereby determining the position of the exon-intron junction.

**19.** A computer readable memory medium storing a program for predicting, identifying or determining a cDNA region of the genome, wherein the program executes the following instructions:

instructions for extracting two non-overlapped sequences, each having at least 10 bases, from a region between each two neighboring homologous regions on the genome of organism 2 on the basis of data on a full

length cDNA sequence of organism 1 or a part thereof, data on the whole genome DNA sequence of organism 2 or a part thereof and a list of the positions of homologous regions between the cDNA sequences of organism 1 and the genome DNA sequence of organism 2, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., regions A1A2, B1B2, ..... are homologous with regions a1a2, b1b2, ....., respectively, and the sequences present on the 5' side and the 3' side of fragment ab are represented by "i" and "j", respectively;

instructions for calculating, with respect to sequences i and j extracted from the region between each two neighboring homologous regions, s(i, j) defined by the following equation:

$$s(i, j) = s(x, yij) - C\{(b1 - j) + (i - a2) - (B1 - A2)\}^2 \qquad \text{(Ia)}$$

wherein

$$s(x, yij) = \max(v(k)) \qquad \text{(II)}$$

$$V(k) = \sum_{p=1}^{myij} M(k+p, p) \qquad (III)$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq 10$,
mj represents the number of bases in sequence j and is $\geq 10$, and
myij represents the number of bases in sequence yij and is $\geq 20$;
instructions for selecting, with respect to the region between each two neighboring homologous regions, a combination of sequences i and j that maximizes s(i, j), thereby determining the positions of exon-intron junction (s); and
instructions for cutting intron sequence(s) out from the genome DNA sequence of organism 2 according to the positions of the exon-intron junction(s) determined, and connecting the remaining pieces to determine the cDNA sequence of organism 2.

**20.** A computer readable memory medium storing a program for predicting, identifying or determining a cDNA region of the genome, wherein the program executes the following instructions:

instructions for searching homologous regions in the genome DNA sequence of organism 2 that are homologous with a full-length cDNA sequence of organism 1 or a part thereof on the basis of data on the full-length cDNA sequence of organism 1 or a part thereof and data on the whole genome DNA sequence of organism 2 or a part thereof;
instructions for making combinations of the homologous regions in the genome DNA sequence of organism 2;
instructions for removing, from the combinations obtained, combinations that cannot exist as cDNA sequences;
instructions for selecting, from the combinations obtained, a combination that gives the widest coverage on the genome DNA sequence of organism 2, thereby making a list of the positions of the homologous regions, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, .....,

respectively;

instructions for selecting two non-overlapped sequences, each having at least 10 bases, from a region between each two neighboring homologous regions, in which region the sequences present on the 5' end side and the 3' end side are represented by "i" and "j", respectively;

instructions for calculating, with respect to sequences i and j extracted from the region between each two neighboring homologous regions, s(i, j) defined by the following equation:

$$s(i, j) = s(x, yij) - C\{(b1 - j) + (i - a2) - (B1 - A2)\}^2 \qquad (Ia)$$

wherein

$$s(x, yij) = \max (v(k)) \qquad (II)$$

$$V(k) = \sum_{p=1}^{myij} M(k+p, p) \qquad (III)$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v(k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq$ 10,

mj represents the number of bases in sequence j and is $\geq$ 10, and

myij represents the number of bases in sequence yij and is $\geq$ 20;

instructions for selecting, with respect to the region between each two neighboring homologous regions, a combination of sequences i and j that maximizes s(i, j), thereby determining the positions of exon-intron junction (s); and

instructions for cutting intron sequence(s) out from the genome DNA sequence of organism 2 according to the positions of the exon-intron junction(s) determined, and connecting the remaining pieces to determine the cDNA sequence of organism 2.

21. The memory medium according to claim 20, wherein the combinations that cannot exist as cDNA sequences are the following:

- a combination in which homologous regions in organism 1 that correspond to two or more homologous regions in organism 2 are the same;
- a combination in which the order of two or more homologous regions in organism 2 is opposite to that of the corresponding homologous regions in organism 1; and
- a combination in which the directions of two or more homologous regions in organism 2 are inverted.

22. The memory medium according to claim 20, wherein the homology search is carried out with a probability of not more than $10^{-50}$ in the instructions for the homology search for the genome region of organism 2.

23. The memory medium according to claim 20, wherein the instructions for the homology search for the genome region of organism 2 comprises instructions for carrying out the homology search by a search system selected from BLAST, LALIGN, ALIGN and FASTA.

24. The memory medium according to claim 19 or 20, further comprising instructions for determining a region that exists 5'-upstream of the homologous region located on the very 5' end side of the genome of organism 2, and a region that exists 3'-downstream of the homologous region located on the very 3' end side of the same.

**25.** The memory medium according to any of claims 17 to 24, wherein v(k) is represented by the following equation.

$$V'(k)=\sum_{p=1}^{myij} M(k+p,p)+max(\sum_{p=1}^{myij}M(k-n+p,p)\times 0.5;n=-6\sim 6) \qquad (VI)$$

**26.** The memory medium according to any of claims 17 to 25, wherein sequences i and j are extracted in accordance with the GT-AG rule in the instructions for extracting sequences i and j.

**27.** The memory medium according to any of claims 17 to 26, wherein mi is 20, mj is 20, and myij is 40 in the instructions for calculating s(i, j).

**28.** The memory medium according to any of claims 17 to 27, wherein organisms 1 and 2 closely relate to each other in terms of the existence and/or homology of genes.

**29.** The memory medium according to claim 28, wherein organisms 1 and 2 are eukaryotes.

**30.** The memory medium according to claim 28, wherein organisms 1 and 2 are mammals.

**31.** The memory medium according to claim 30, wherein organism 1 is a mouse and organism 2 is a human.

**32.** The memory medium according to claim 30, wherein organism 1 is a human and organism 2 is a mouse.

**33.** A method for predicting, identifying or determining an exon-intron junction in a gene region of the genome, comprising the steps of:

preparing data on a full-length cDNA sequence of organism 1 or a part thereof (fragment AB) and the corresponding gene region of the genome of organism 2 (fragment ab);
extracting two non-overlapped sequences, each having at least 10 bases, from fragment ab, wherein the sequences present on the 5' side and the 3' side of fragment ab are represented by "i" and "j", respectively;
calculating, with respect to sequences i and j extracted, s(i, j) defined by the following equation :

$$s(i, j) = s(x, yij) - C\{(b - j) + (i - a) - (B - A)\}^2 \qquad (I)$$

wherein

$$s(x, yij) = max (v(k)) \qquad (II)$$

$$V(k)=\sum_{p=1}^{myij}M(k+p,p) \qquad (III)$$

(b - j) represents the number of bases between the 3' end of the gene region of organism 2 and the 5' end of sequence j,
(i - a) represents the number of bases between the 5' end of the gene region of organism 2 and the 3' end of sequence i,
(B - A) represents the number of bases in the cDNA of organism 1,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20;
selecting a combination of sequences i and j that maximizes s(i, j); and
determining the position of the exon-intron junction.

**34.** A method for predicting, identifying or determining an exon-intron junction in a gene region of the genome, comprising the steps of:

preparing data on a full-length cDNA sequence of organism 1 or a part thereof (fragment AB) and the corresponding gene region of the genome of organism 2 (fragment ab), wherein the full-length cDNA sequence of organism 1 or a part thereof and the gene region of the genome of organism 2 have homologous regions at their end parts, homologous regions in the cDNA sequence of organism 1 are represented by A1A2 and B1B2, homologous regions in the gene region of the genome of organism 2 are represented by a1a2 and b1b2, and regions A1A2 and B1B2 are homologous with regions a1a2 and b1b2, respectively;
extracting two non-overlapped sequences, each having at least 10 bases, from a region between a1a2 and b1b2 in the gene region of the genome of organism 2, wherein the sequences present on the 5' end side and the 3' end side fragment ab are represented by "i" and "j", respectively;
calculating, with respect to sequences i and j extracted, s(i, j) defined by the following equation:

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is $\geq$ 10,
mj represents the number of bases in sequence j and is $\geq$ 10, and
myij represents the number of bases in sequence yij and is $\geq$ 20;
selecting a combination of sequences i and j that maximizes s(i, j); and
determining the position of the exon-intron junction.

**35.** A method for predicting, identifying or determining a cDNA region of the genome, comprising the steps of:

preparing an input part in which data on a full-length cDNA sequence of organism 1 or a part thereof, data on the whole genome DNA sequence of organism 2 or a part thereof and a list of the positions of homologous regions between the cDNA sequence of organism 1 and the genome DNA sequence of organism 2, wherein the cDNA sequence of organism 1 or a part thereof and the gene region of the genome of organism 2 have two or more homologous regions, homologous regions in the cDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively; extracting two non-overlapped sequences, each having at least 10 bases, from a region between each two neighboring homologous regions, in which region the sequences present on the 5' end side and the 3' end side are represented by "i" and "j", respectively;

calculating ,with respect to sequences i and j extracted from the region between each two neighboring homologous regions, s(i, j) defined by the following equation :

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,

(i - a2) represents the number of bases between the 5' end of region a1a2 and the 3' end of sequence i,

(B1 - A2) represents the number of bases between the 3' end of region A1A2 and the 5' end of region B1B2,

C is a proportionality constant from 0 to 10,

v( k) represents an overlap score between x and yij, wherein x is the cDNA sequence of organism 1, yij is a fragment composed of sequences i and j that are connected, and k is an integer of 1 to myij,

M represents a matrix of x and yij, M(a, b) = 1 when a base in position "a" for x is the same base as in position "b" for yij, and M(a, b) = 0 when a base in position "a" for x is not the same base as in position "b" for yij,

mi represents the number of bases in sequence i and is $\geq 10$,

mj represents the number of bases in sequence j and is $\geq 10$, and

myij represents the number of bases in sequence yij and is $\geq 20$;

selecting a combination of sequences i and j that maximizes s(i, j) with respect to the region between each two neighboring homologous regions;

determining the position of the exon-intron junction;

cutting out intron sequences from the genome DNA sequence of organism 2 according to the positions of the exon-intron junctions determined; and

connecting the remaining sequences, thereby determining the cDNA sequence of organism 2.

**36.** A method for predicting, identifying or determining a cDNA region of the genome, comprising the steps of:

preparing data on a full-length cDNA sequence of organism 1 or a part thereof and data on the whole genome DNA sequence of organism 2 or a part thereof;

searching homologous regions in the genome DNA sequence of organism 2 that are homologous with the full-length cDNA sequence of organism 1 or a part thereof;

making combinations of the homologous regions in the genome DNA sequence of organism 2;

removing combinations that cannot exist as cDNA sequences from the combinations obtained;

selecting a combination that gives the widest coverage on the genome DNA from the remaining combinations, thereby making a list of the positions of the homologous regions, wherein the cDNA sequence of organism 1 or a part thereof and the gene region of the genome of organism 2 have two or more homologous regions, homologous regions in the CDNA sequence of organism 1 are represented by A1A2, B1B2, ....., homologous regions in the gene region of the genome of organism 2 are represented by a1a2, b1b2, ....., and regions A1A2, B1B2, ..... are homologous with regions a1a2, b1b2, ....., respectively;

extracting two non-overlapped sequences, each having at least 10 bases, from a region between each two neighboring homologous regions, in which region the sequences present on the 5' end side and the 3' end side are represented by "i" and "j", respectively;

calculating, with respect to sequences i and j extracted from the region between each two neighboring homologous regions,s(i, j) defined by the following equation:

$$s(i, j) = s(x, yij) - C\{(b1 - j) + (i - a2) - (B1 - A2)\}^2 \qquad \text{(Ia)}$$

wherein

$$s(x, yij) = \max (v(k)) \qquad \text{(II)}$$

$$V(k) = \sum_{p=1}^{myij} M(k+p, p) \qquad \text{(III)}$$

(b1 - j) represents the number of bases between the 5' end of region b1b2 and the 5' end of sequence j,
(i - a2) represents the number of bases between the 5' end of region a1a2 and the 3' end of sequence i,
(B1 - A2) represents the number of bases between the 3' end of region A1A2 and the 5' end of region B1B2,
C is a proportionality constant from 0 to 10,
v(k) represents an overlap score between x and yij, wherein x is the cDNA sequence of organism 1, yij is a fragment composed of sequences i and j that are connected, and k is an integer of 1 to myij,
M represents a matrix of x and yij, M(a, b) = 1 when a base in position "a" for x is the same base as in position "b" for yij, and M(a, b) = 0 when a base in position "a" for x is not the same base as in position "b" for yij,
mi represents the number of bases in sequence i and is ≥ 10,
mj represents the number of bases in sequence j and is ≥ 10, and
myij represents the number of bases in sequence yij and is ≥ 20;
selecting a combination of sequences i and j that maximizes s(i, j) with respect to the region between each two neighboring homologous regions;
determining the position of the exon-intron junction;
cutting out intron sequences from the genome DNA sequence of organism 2 according to the positions of the exon-intron junctions determined; and
connecting the remaining sequences, thereby determining the cDNA sequence of organism 2.

**37.** The method according to claim 36, wherein the combinations that cannot exist as cDNA sequences are as follows:

- a combination in which homologous regions in organism 1 that correspond to two or more homologous regions in organism 2 are the same;
- a combination in which the order of two or more homologous regions in organism 2 is opposite to that of the corresponding homologous regions in organism 1; and
- a combination in which the directions of two or more homologous regions in organism 2 are inverted.

**38.** The method according to claim 36, wherein the homology search is carried out with a probability of not more than $10^{-50}$ in the homology search step for the genome region of organism 2.

**39.** The method according to claim 36, wherein the homology search step for the genome region of organism 2 comprises a step of carrying out the homology search by a search system selected from BLAST, LALIGN, ALIGN and FASTA.

**40.** The method according to claim 35 or 36, further comprising a step of determining a region that exists 5'-upstream of the homologous region located on the very 5' end side of the genome of organism 2, and a region that exists 3'-downstream of the homologous region located on the very 3' end side of the same.

**41.** The method according to any of claims 33 to 40, wherein v(k) is represented by the following equation.

$$V'(k)=\sum_{p=1}^{myij} M(k+p,p)+\max(\sum_{p=1}^{myij} M(k-n+p,p)\times 0.5; n=-6\sim 6) \qquad (VI)$$

42. The method according to any of claims 33 to 41, wherein sequences i and j are extracted in accordance with the GT-AG rule in the step of extracting sequences i and j.

43. The method according to any of claims 33 to 42, wherein mi is 20, mj is 20, and myij is 40 in the step of calculating s(i, j).

44. The method according to any of claims 33 to 43, wherein organisms 1 and 2 closely relate to each other in terms of the existence and/or homology of genes.

45. The method according to claim 44, wherein organisms 1 and 2 are eukaryotes.

46. The method according to claim 44, wherein organisms 1 and 2 are mammals.

47. The method according to claim 46, wherein organism 1 is a mouse and organism 2 is a human.

48. The method according to claim 46, wherein organism 1 is a human and organism 2 is a mouse.

INPUT PART

OPERATION PART

JUNCTION
DETERMINATION
PART

OUTPUT PART

# F I G. I

```
┌─────────────────┐
│   INPUT PART    │
└─────────────────┘
         │
         ▼
┌─────────────────┐         ┌─────────────────┐
│  OPERATION PART │ ───────▶│   MEMORY PART   │
│                 │ ◀─────── │                 │
└─────────────────┘         └─────────────────┘
         │
         ▼
┌─────────────────┐
│    JUNCTION     │
│  DETERMINATION  │
│     PART        │
└─────────────────┘
         │
         ▼
┌─────────────────┐
│   OUTPUT PART   │
└─────────────────┘
```

# F I G. 2

```
┌─────────────────────┐
│                     │
│     INPUT PART      │
│                     │
└─────────────────────┘
           │
           ▼
┌─────────────────────┐
│                     │
│   OPERATION PART    │
│                     │
└─────────────────────┘
           │
           ▼
┌─────────────────────┐
│     JUNCTION        │
│   DETERMINATION     │
│     PART            │
└─────────────────────┘
           │
           ▼
┌─────────────────────┐
│   END PORTION       │
│   DETERMINATION     │
│     PART            │
└─────────────────────┘
           │
           ▼
┌─────────────────────┐
│                     │
│    OUTPUT PART      │
│                     │
└─────────────────────┘
```

# F I G. 3

```
┌─────────────────────┐
│                     │
│     INPUT PART      │
│                     │
└─────────────────────┘
           │
           ▼
┌─────────────────────┐          ┌─────────────────────┐
│                     │─────────▶│                     │
│   OPERATION PART    │          │     MEMORY PART     │
│                     │◀─────────│                     │
└─────────────────────┘          └─────────────────────┘
           │
           ▼
┌─────────────────────┐
│     JUNCTION        │
│   DETERMINATION     │
│     PART            │
└─────────────────────┘
           │
           ▼
┌─────────────────────┐
│   END PORTION       │
│   DETERMINATION     │
│     PART            │
└─────────────────────┘
           │
           ▼
┌─────────────────────┐
│                     │
│    OUTPUT PART      │
│                     │
└─────────────────────┘
```

F I G. 4

FIG. 5

```
┌─────────────────────┐
│     INPUT PART      │
└─────────────────────┘
           │
           ▼
┌─────────────────────┐
│  HOMOLOGY SEARCH    │
│  PART               │
└─────────────────────┘
           │
           ▼
┌─────────────────────┐
│  POSITION LIST      │
│  MAKING PART        │
└─────────────────────┘
           │
           ▼
┌─────────────────────┐       ┌─────────────────────┐
│                     │──────▶│                     │
│  OPERATION PART     │       │  MEMORY PART        │
│                     │◀──────│                     │
└─────────────────────┘       └─────────────────────┘
           │
           ▼
┌─────────────────────┐
│  JUNCTION           │
│  DETERMINATION      │
│  PART               │
└─────────────────────┘
           │
           ▼
┌─────────────────────┐
│     OUTPUT PART     │
└─────────────────────┘
```

# FIG. 6

INPUT PART

↓

HOMOLOGY SEARCH
PART

↓

POSITION LIST
MAKING PART

↓

OPERATION PART

↓

JUNCTION
DETERMINATION
PART

↓

END PORTION
DETERMINATION
PART

↓

OUTPUT PART

# F I G. 7

F I G. 8

INSTRUCTIONS FOR EXTRACTING SEQUENCES i AND j FROM THE GENOME DNA SEQUENCE OF ORGANISM 2 ON THE BASIS OF A cDNA SEQUENCE OF ORGANISM 1 AND THE GENOME DNA SEQUENCE OF ORGANISM 2.

INSTRUCTIONS FOR CALCULATING s(i, j) WITH RESPECT TO SEQUENCES i AND j EXTRACTED.

INSTRUCTIONS FOR SELECTING A COMBINATION OF SEQUENCES i AND j THAT MAXIMIZES s(i, j), THEREBY DETERMINING THE POSITION OF AN EXON-INTRON JUNCTION.

# FIG.9

INSTRUCTIONS FOR EXTRACTING
SEQUENCES i AND j, ON THE BASIS OF
A cDNA SEQUENCE OF ORGANISM 1,
THE GENOME DNA SEQUENCE OF
ORGANISM 2 AND A LIST OF THE
POSITIONS OF HOMOLOGOUS REGIONS,
FROM A REGION BETWEEN EACH TWO
NEIGHBORING HOMOLOGOUS REGIONS
IN THE GENOME DNA SEQUENCE OF
ORGANISM 2.

INSTRUCTIONS FOR CALCULATING s(i, j)
WITH RESPECT TO SEQUENCES i AND j
EXTRACTED FROM A REGION BETWEEN
EACH TWO NEIGHBORING HOMOLOGOUS
REGIONS.

INSTRUCTIONS FOR SELECTING,
WITH RESPECT TO A REGION BETWEEN
EACH TWO NEIGHBORING HOMOLOGOUS
REGIONS, A COMBINATION OF
SEQUENCES i AND j THAT MAXIMIZES
s(i, j), THEREBY DETERMINING THE
POSITIONS OF EXON-INTRON
JUNCTION(S)

INSTRUCTIONS FOR CUTTING INTRONS
OUT ACCORDING TO THE POSITIONS OF
THE EXON-INTRON JUNCTION(S)
DETERMINED, THEREBY DETERMINING
THE cDNA SEQUENCE OF ORGANISM 2.

# FIG.10

INSTRUCTIONS FOR EXTRACTING SEQUENCES i AND j, ON THE BASIS OF A cDNA SEQUENCE OF ORGANISM 1, THE GENOME DNA SEQUENCE OF ORGANISM 2 AND A LIST OF THE POSITIONS OF HOMOLOGOUS REGIONS, FROM A REGION BETWEEN EACH TWO NEIGHBORING HOMOLOGOUS REGIONS IN THE GENOME DNA SEQUENCE OF ORGANISM 2.

INSTRUCTIONS FOR CALCULATING $s(i, j)$ WITH RESPECT TO SEQUENCES i AND j EXTRACTED FROM A REGION BETWEEN EACH TWO NEIGHBORING HOMOLOGOUS REGIONS.

INSTRUCTIONS FOR SELECTING, WITH RESPECT TO A REGION BETWEEN EACH TWO NEIGHBORING HOMOLOGOUS REGIONS, A COMBINATION OF SEQUENCES i AND j THAT MAXIMIZES $s(i, j)$, THEREBY DETERMINING THE POSITIONS OF EXON-INTRON JUNCTION(S)

INSTRUCTIONS FOR DETERMINING THE 5' END REGION AND THE 3' END REGION.

INSTRUCTIONS FOR CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.11

INSTRUCTIONS FOR SEARCHING REGIONS IN THE GENOME DNA SEQUENCE OF ORGANISM 2 THAT ARE HOMOLOGOUS WITH A cDNA SEQUENCE OF ORGANISM 1.

↓

INSTRUCTIONS FOR MAKING COMBINATIONS OF THE HOMOLOGOUS REGIONS DETERMINED.

↓

INSTRUCTIONS FOR REMOVING, FROM THE OBTAINED COMBINATIONS OF THE HOMOLOGOUS REGIONS, COMBINATIONS THAT CANNOT EXIST AS cDNA SEQUENCES.

↓

INSTRUCTIONS FOR SELECTING A COMBINATION THAT COVERS THE WIDEST RANGE ON THE GENOME DNA, THEREBY MAKING A LIST OF THE POSITIONS OF THE HOMOLOGOUS REGIONS SELECTED.

↓

INSTRUCTIONS FOR EXTRACTING SEQUENCES i AND j FROM A REGION BETWEEN EACH TWO NEIGHBORING HOMOLOGOUS REGIONS IN THE GENOME DNA SEQUENCE OF ORGANISM 2.

INSTRUCTIONS FOR CALCULATING s(i, j) WITH RESPECT TO SEQUENCES i AND j EXTRACTED FROM A REGION BETWEEN EACH TWO NEIGHBORING HOMOLOGOUS REGIONS.

↓

INSTRUCTIONS FOR SELECTING, WITH RESPECT TO A REGION BETWEEN EACH TWO NEIGHBORING HOMOLOGOUS REGIONS, A COMBINATION OF SEQUENCES i AND j THAT MAXIMIZES s(i, j), THEREBY DETERMINING THE POSITIONS OF EXON-INTRON JUNCTION(S)

↓

INSTRUCTIONS FOR CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.12

INSTRUCTIONS FOR SEARCHING REGIONS IN THE GENOME DNA SEQUENCE OF ORGANISM 2 THAT ARE HOMOLOGOUS WITH A cDNA SEQUENCE OF ORGANISM 1.

↓

INSTRUCTIONS FOR MAKING COMBINATIONS OF THE HOMOLOGOUS REGIONS DETERMINED.

↓

INSTRUCTIONS FOR REMOVING, FROM THE OBTAINED COMBINATIONS OF THE HOMOLOGOUS REGIONS, COMBINATIONS THAT CANNOT EXIST AS cDNA SEQUENCES.

↓

INSTRUCTIONS FOR SELECTING A COMBINATION THAT COVERS THE WIDEST RANGE ON THE GENOME DNA, THEREBY MAKING A LIST OF THE POSITIONS OF THE HOMOLOGOUS REGIONS SELECTED.

↓

INSTRUCTIONS FOR EXTRACTING SEQUENCES i AND j FROM A REGION BETWEEN EACH TWO NEIGHBORING HOMOLOGOUS REGIONS IN THE GENOME DNA SEQUENCE OF ORGANISM 2.

INSTRUCTIONS FOR CALCULATING s(i, j) WITH RESPECT TO SEQUENCES i AND j EXTRACTED FROM A REGION BETWEEN EACH TWO NEIGHBORING HOMOLOGOUS REGIONS.

↓

INSTRUCTIONS FOR SELECTING, WITH RESPECT TO A REGION BETWEEN EACH TWO NEIGHBORING HOMOLOGOUS REGIONS, A COMBINATION OF SEQUENCES i AND j THAT MAXIMIZES s(i, j), THEREBY DETERMINING THE POSITIONS OF EXON-INTRON JUNCTION(S)

↓

INSTRUCTIONS FOR DETERMINING THE 5' END REGION AND THE 3' END REGION.

↓

INSTRUCTIONS FOR CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.13

STEP OF PREPARING A cDNA OF
ORGANISM 1 AND THE GENOME DNA OF
ORGANISM 2.

STEP OF EXTRACTING SEQUENCES
i AND j FROM THE GENOME DNA
SEQUENCE OF ORGANISM 2 ON THE
BASIS OF THE cDNA OF ORGANISM 1 AND
THE GENOME DNA OF ORGANISM 2.

STEP OF CALCULATING $s(i, j)$ WITH
RESPECT TO SEQUENCES i AND j
EXTRACTED.

STEP OF SELECTING A COMBINATION
OF SEQUENCES i AND j THAT MAXIMIZES
$s(i, j)$, THEREBY DETERMINING THE
POSITION OF THE EXON-INTRON
JUNCTION.

# FIG.14

STEP OF PREPARING A cDNA
SEQUENCE OF ORGANISM 1, THE GENOME
DNA SEQUENCE OF ORGANISM 2 AND A
LIST OF THE POSITIONS OF HOMOLOGOUS
REGIONS.

STEP OF EXTRACTING SEQUENCES
i AND j, ON THE BASIS OF THE cDNA
SEQUENCE OF ORGANISM 1, THE GENOME
DNA SEQUENCE OF ORGANISM 2 AND THE
LIST OF THE POSITIONS OF HOMOLOGOUS
REGIONS, FROM A REGION BETWEEN EACH
TWO NEIGHBORING HOMOLOGOUS
REGIONS IN THE GENOME DNA SEQUENCE
OF ORGANISM 2.

STEP OF CALCULATING s(i, j) WITH
RESPECT TO SEQUENCES i AND j
EXTRACTED FROM A REGION BETWEEN
EACH TWO NEIGHBORING HOMOLOGOUS
REGIONS.

STEP OF SELECTING, WITH RESPECT
TO A REGION BETWEEN EACH TWO
NEIGHBORING HOMOLOGOUS REGIONS,
A COMBINATION OF SEQUENCES i AND j
THAT MAXIMIZES s(i, j), THEREBY
DETERMINING THE POSITIONS OF
EXON-INTRON JUNCTION(S)

STEP OF CUTTING INTRONS OUT
ACCORDING TO THE POSITIONS OF THE
EXON-INTRON JUNCTION(S) DETERMINED,
THEREBY DETERMINING THE cDNA
SEQUENCE OF ORGANISM 2.

# FIG.15

STEP OF PREPARING A cDNA SEQUENCE OF ORGANISM 1, THE GENOME DNA SEQUENCE OF ORGANISM 2 AND A LIST OF THE POSITIONS OF HOMOLOGOUS REGIONS.

STEP OF EXTRACTING SEQUENCES i AND j, ON THE BASIS OF THE cDNA SEQUENCE OF ORGANISM 1, THE GENOME DNA SEQUENCE OF ORGANISM 2 AND THE LIST OF THE POSITIONS OF HOMOLOGOUS REGIONS, FROM A REGION BETWEEN EACH TWO NEIGHBORING HOMOLOGOUS REGIONS IN THE GENOME DNA SEQUENCE OF ORGANISM 2.

STEP OF CALCULATING s(i, j) WITH RESPECT TO SEQUENCES i AND j EXTRACTED FROM A REGION BETWEEN EACH TWO NEIGHBORING HOMOLOGOUS REGIONS.

STEP OF SELECTING, WITH RESPECT TO A REGION BETWEEN EACH TWO NEIGHBORING HOMOLOGOUS REGIONS, A COMBINATION OF SEQUENCES i AND j THAT MAXIMIZES s(i, j), THEREBY DETERMINING THE POSITIONS OF EXON-INTRON JUNCTION(S)

STEP OF DETERMINING THE 5' END REGION AND THE 3' END REGION.

STEP OF CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.16

STEP OF PREPARING A cDNA SEQUENCE OF ORGANISM 1 AND THE GENOME DNA SEQUENCE OF ORGANISM 2.

STEP OF SEARCHING REGIONS IN THE GENOME DNA SEQUENCE OF ORGANISM 2 THAT ARE HOMOLOGOUS WITH THE cDNA SEQUENCE OF ORGANISM 1.

STEP OF MAKING COMBINATIONS OF THE HOMOLOGOUS REGIONS DETERMINED.

STEP OF REMOVING, FROM THE COMBINATIONS OF THE HOMOLOGOUS REGIONS OBTAINED, COMBINATIONS THAT CANNOT EXIST AS cDNA SEQUENCES.

STEP OF SELECTING A COMBINATION THAT COVERS THE WIDEST RANGE ON THE GENOME DNA, THEREBY MAKING A LIST OF THE POSITIONS OF THE HOMOLOGOUS REGIONS SELECTED.

STEP OF EXTRACTING SEQUENCES i AND j FROM A REGION BETWEEN EACH TWO NEIGHBORING HOMOLOGOUS REGIONS IN THE GENOME DNA SEQUENCE OF ORGANISM 2.

STEP OF CALCULATING $s(i, j)$ WITH RESPECT TO SEQUENCES i AND j EXTRACTED FROM A REGION BETWEEN EACH TWO NEIGHBORING HOMOLOGOUS REGIONS.

STEP OF SELECTING, WITH RESPECT TO A REGION BETWEEN EACH TWO NEIGHBORING HOMOLOGOUS REGIONS, A COMBINATION OF SEQUENCES i AND j THAT MAXIMIZES $s(i, j)$, THEREBY DETERMINING THE POSITIONS OF EXON-INTRON JUNCTION(S)

STEP OF CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.17

STEP OF PREPARING A cDNA SEQUENCE OF ORGANISM 1 AND THE GENOME DNA SEQUENCE OF ORGANISM 2.

STEP OF SEARCHING REGIONS IN THE GENOME DNA SEQUENCE OF ORGANISM 2 THAT ARE HOMOLOGOUS WITH THE cDNA SEQUENCE OF ORGANISM 1.

STEP OF MAKING COMBINATIONS OF THE HOMOLOGOUS REGIONS DETERMINED.

STEP OF REMOVING, FROM THE COMBINATIONS OF THE HOMOLOGOUS REGIONS OBTAINED, COMBINATIONS THAT CANNOT EXIST AS cDNA SEQUENCES.

STEP OF SELECTING A COMBINATION THAT COVERS THE WIDEST RANGE ON THE GENOME DNA, THEREBY MAKING A LIST OF THE POSITIONS OF THE HOMOLOGOUS REGIONS SELECTED.

STEP OF EXTRACTING SEQUENCES i AND j FROM A REGION BETWEEN EACH TWO NEIGHBORING HOMOLOGOUS REGIONS IN THE GENOME DNA SEQUENCE OF ORGANISM 2.

STEP OF CALCULATING $s(i, j)$ WITH RESPECT TO SEQUENCES i AND j EXTRACTED FROM A REGION BETWEEN EACH TWO NEIGHBORING HOMOLOGOUS REGIONS.

STEP OF SELECTING, WITH RESPECT TO A REGION BETWEEN EACH TWO NEIGHBORING HOMOLOGOUS REGIONS, A COMBINATION OF SEQUENCES i AND j THAT MAXIMIZES $s(i, j)$, THEREBY DETERMINING THE POSITIONS OF EXON-INTRON JUNCTION(S)

STEP OF DETERMINING THE 5' END REGION AND THE 3' END REGION.

STEP OF CUTTING INTRONS OUT ACCORDING TO THE POSITIONS OF THE EXON-INTRON JUNCTION(S) DETERMINED, THEREBY DETERMINING THE cDNA SEQUENCE OF ORGANISM 2.

FIG.18

FIG. 19

FIG. 20

ORGANISM 1
cDNA

5'        I        II        III        IV        3'

ORGANISM 2
GENOME DNA

5'        3'

I        II        III        IV

# F I G. 2I

cDNA SEQUENCE① OF ORGANISM 1 IS READ IN.

GENOME DNA SEQUENCE② OF ORGANISM 2 IS READ IN.

LIST③ OF HOMOLOGOUS REGIONS IS READ IN.

i = 1~ N

ON THE BASIS OF LIST③, SPLICE SITE CANDIDATES IN EACH HOMOLOGOUS REGION IN GENOME DNA SEQUENCE② ARE CALCULATED, AND STORED IN THE LIST (STORAGE IN THE DATA SEGMENT).

# FIG.22

I = 1~N

i = 1~ $n^3$( I )

j = 1~ $n^5$( I-1 )

FROM GENOME DNA SEQUENCE②, A SEQUENCE OF 20 BASE PAIRS UPSTREAM OF $m^3$(I, i) AND A SEQUENCE OF 20 BASE PAIRS DOWNSTREAM OF $m^5$(I - 1, j) ARE TAKEN, AND CONNECTED TO OBTAIN yij.

cDNA SEQUENCE① IS TAKEN AS x.

M, v(k) AND s ARE CALCULATED WITH RESPECT TO yij AND x.

WITH RESPECT TO EACH I, $m^5$(I, j) AND $m^3$(I, i) THAT MAXIMIZES ARE SELECTED.

SEQUENCES ARE CUT OUT FROM GENOME DNA SEQUENCE② AT SITES $m^5$ AND $m^3$ SELECTED ABOVE, AND THE REMAINING PIECES ARE CONNECTED TO OUTPUT THE cDNA SEQUENCE OF ORGANISM 2.

# FIG.23

ORGANISM 1 cDNA

(1500 bp) 5' ⊢ | | ACCATGG | | | AATAAA | | | -3'

ORGANISM 2 GENOME DNA (150 kbp)

REGION 1 ACCATG (300 bp)

REGION 2 AATAAA (900 bp)

REGION 3 ACCATG (600 bp)

REGION 4 TTTATT (900 bp)

F I G. 24

FIG. 25

FIG. 26

| INTERNATIONAL SEARCH REPORT | International application No. |
|---|---|
| | PCT/JP00/08402 |

| A. CLASSIFICATION OF SUBJECT MATTER |
|---|
| Int.Cl$^7$ G06F17/00, G01N33/50 |
| According to International Patent Classification (IPC) or to both national classification and IPC |

| B. FIELDS SEARCHED |
|---|
| Minimum documentation searched (classification system followed by classification symbols) |
| Int.Cl$^7$ G06F17/00, G01N33/50 |
| Documentation searched other than minimum documentation to the extent that such documents are included in the fields searched |
| Jitsuyo Shinan Koho 1926-1996 Jitsuyo Shinan Toroku Koho 1996-2000 Kokai Jitsuyo Shinan Koho 1971-2000 Toroku Jitsuyo Shinan Koho 1994-2000 |
| Electronic data base consulted during the international search (name of data base and, where practicable, search terms used) JICST FILE, "(INTRON*JUNCTION)+(SPLICING)+(GENE*REGION DECISION)" (in Japanese) INSPEC,"INTRON*EXON" |

| C. DOCUMENTS CONSIDERED TO BE RELEVANT |
|---|

| Category* | Citation of document, with indication, where appropriate, of the relevant passages | Relevant to claim No. |
|---|---|---|
| A | CRAIK, C. S. et al. Splice Junctions : Association with Variation in Protein Structure. SCINECE., 10 June 1983 , Vol.220, No.4602, pages 1125-1129 | 1-48 |
| A | Takeo SEKIYA, et al., "Hatsugan no Bunshi Kikou ni Kansuru Kenkyu: Gan Kanren Idenshi no Atarashii Joho no Chiryou e no Ouyou," (Japan) 1996 nendo Kouseishou Gan Kokufuku Shin 10 kanen Senryaku Project Kenkyu Houkokusho, 31 March 1996, pages 19-27. | 1-48 |
| A | JP, 11-187885, A (Mitsubishi Kagaku Bio-Clinical Lab. Inc.), 13 July, 1999 (13.07.99), Full text (Family: none) | 1-48 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 March, 2001 (07.03.01) | 21 March, 2001 (21.03.01) |

| Name and mailing address of the ISA/ | Authorized officer |
|---|---|
| Japanese Patent Office | |
| Facsimile No. | Telephone No. |

Form PCT/ISA/210 (second sheet) (July 1992)